# EUROPEAN PATENT SPECIFICATION

(11) **EP 3 726 599 B1**
(45) Date of publication and mention of the grant of the patent: **23.03.2022**
(21) Application number: 19169221.9
(22) Date of filing: 15.04.2019
(51) Int. Cl.: H01L 51/54, C07D 219/04, C07D 221/18

(54) **COMPOUND, ORGANIC SEMICONDUCTING MATERIAL COMPRISING THE SAME, ORGANIC ELECTRONIC DEVICE COMPRISING THE SAME, AND DISPLAY DEVICE AND LIGHTING DEVICE COMPRISING THE SAME**
VERBINDUNG, ORGANISCHES HALBLEITENDES MATERIAL DAMIT, ORGANISCHE ELEKTRONISCHE VORRICHTUNG DAMIT SOWIE ANZEIGEVORRICHTUNG UND BELEUCHTUNGSVORRICHTUNG DAMIT
COMPOSÉ, MATÉRIAU SEMICONDUCTEUR ORGANIQUE LE COMPRENANT, DISPOSITIF ÉLECTRONIQUE ORGANIQUE LE COMPRENANT, DISPOSITIF D'AFFICHAGE ET DISPOSITIF D'ÉCLAIRAGE LE COMPRENANT

(43) Date of publication of application: 21.10.2020
(73) Proprietor: Novaled GmbH, 01099 Dresden (DE)
(72) Inventor: CARDINALI, François, 01099 Dresden (DE); SCHULZE, Benjamin, 01099 Dresden (DE); GALAN GARCIA, Elena, 01099 Dresden (DE); PAVICIC, Domagoj, 01099 Dresden (DE); RUNGE, Steffen, 01099 Dresden (DE); SCHOLZ, Johannes, 01099 Dresden (DE)
(74) Representative: Bittner, Thomas L.

(56) References cited:
- EP-A1- 3 312 171
- WO-A1-2011/154131
- US-A1- 2015 364 697

## Description

The present invention relates to a compound an organic semiconducting material comprising the same and an organic electronic device comprising the same. The invention further relates to a display device or a lighting device comprising the organic electronic device.

### BACKGROUND ART

Organic light-emitting diodes (OLEDs), which are self-emitting devices, have a wide viewing angle, excellent contrast, quick response, high brightness, excellent driving voltage characteristics, and color reproduction. A typical OLED includes an anode, a hole transport layer (HTL), an emission layer (EML), an electron transport layer (ETL), and a cathode, which are sequentially stacked on a substrate. In this regard, the HTL, the EML, and the ETL are thin films formed essentially by organic and / or organometallic compounds.

When a voltage is applied to the anode and the cathode, holes injected from the anode electrode move to the EML, via the HTL, and electrons injected from the cathode electrode move to the EML, via the ETL. The holes and electrons mainly recombine in the EML to generate excitons. When the excitons drop from an excited state to a ground state, light is emitted. The injection and flow of holes and electrons should be balanced, so that an OLED having the above-described structure has excellent efficiency.

Important role among OLED devices used in state-of-art displays play devices comprising electron transport layers doped with metal salts or metal complexes, such as e.g. lithium 8-hydroxyquinolinolate (LiQ), to improve the performance of the device.

Dibenzo[c,h]acridine compounds disclosed e.g. in WO2011/154131, WO2013/079217 or WO2018/077689 already form an important class of matrix compounds which may be used in organic semiconducting materials, preferably in materials designed for electron injection, electron transport or electron generating layers in OLEDs. There is, however, a continuous demand for new materials having improved properties and/or providing the engineers designing organic electronic devices with broader options for selection of a suitable material.

US 2015/364697 A1 discloses an organic light-emitting device including: a first electrode; a second electrode; an emission layer between the first electrode and the second electrode; a hole transport region between the first electrode and the emission layer; and an electron transport region between the second electrode and the emission layer, wherein the hole transport region includes a first compound represented by Formula 1.

WO 2011/154131 A1 discloses an electronic device comprising at least one organic semiconducting material.

It is, therefore, the object of the present invention to provide organic semiconducting materials, respectively compounds for use in such materials suitable to improve the operational voltage and efficiency of organic electronic devices using the same and/or to improve the design freedom with respect to such devices.

The above problems are solved in accordance with the subject matter of the independent claims. Further embodiments result from the subclaims.

This object is, first of all, achieved by an organic semiconducting material comprising a compound represented by the following formula (Ia) 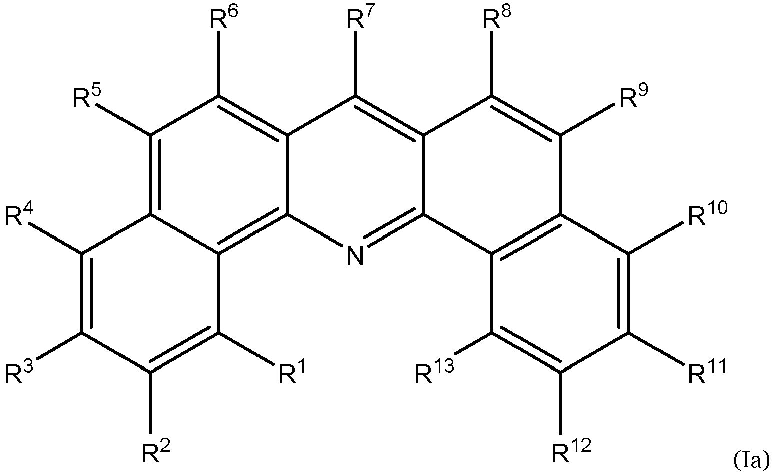
wherein R¹ to R¹³ are independently selected from the group consisting of H, D, F, Cl, Br, I, substituted or unsubstituted C₁ to C₁₈ alkyl, substituted or unsubstituted C₆ to C₄₂ aryl, substituted or unsubstituted C₃ to C₄₂ heteroaryl,
wherein two or more groups selected from R¹ to R¹³ may be linked to form a non-aromatic ring;
wherein at least one of R¹ to R¹³ is a group substituted with at least one CN;
wherein the one or more substituent(s), if present in one of the groups R¹ to R¹³, are independently selected from the group consisting of D, F, Cl, Br, I, C₁ to C₁₈ alkyl, C₆ to C₃₆ aryl, C₆ to C₄₂ heteroaryl, R¹⁴R¹⁵P=O with R¹⁴ and R¹⁵ independently being C₁ to C₁₈ alkyl or C₆ to C₂₄ aryl, and CN,
wherein the compounds represented by the following formulas (1) to (5) are excluded

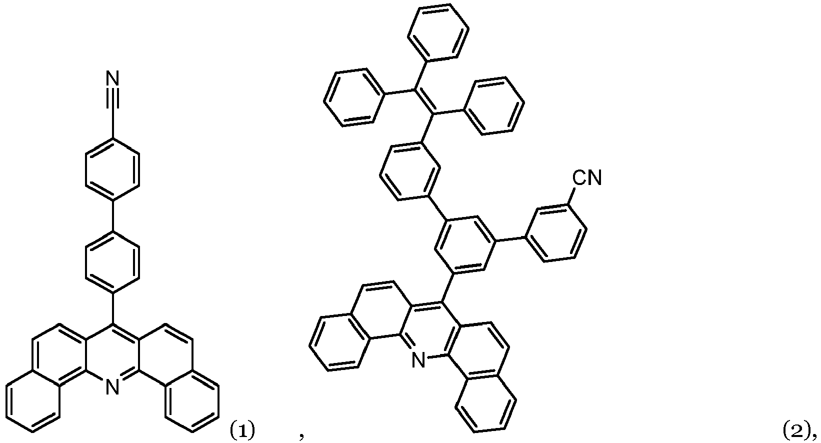

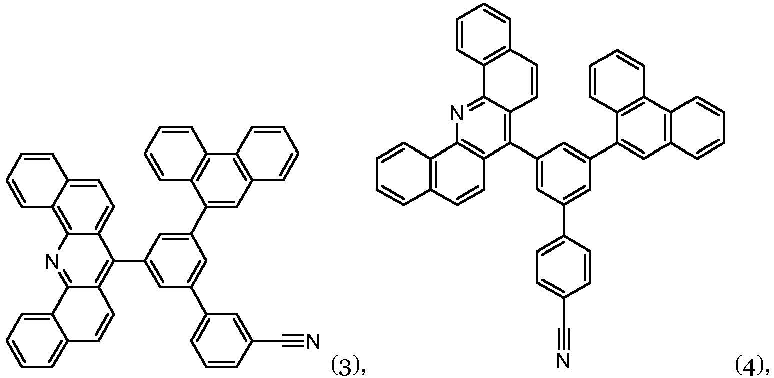

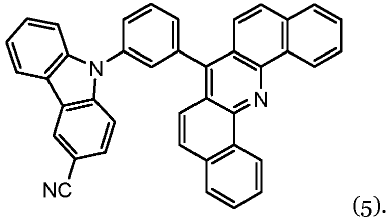

The inventors have surprisingly found that combining in one compound a dibenzo[c,h]acridine structural moiety with one or more CN groups is a viable way for designing new organic semiconducting matrix materials improving design freedom for new organic electronic devices, including OLEDs and OLED displays. The new compounds are particularly suitable for design of electron injection, electron transport and/or electron generating materials.

It was furthermore found by the inventors that particular embodiments falling within the scope of the above generic definition of inventive compounds are particularly advantageous for improving operational voltage and/or efficiency of organic devices using such compounds in organic semiconducting materials thereof. Respective embodiments will be described in the following. The inventors have further found that combination of two or more of such embodiments is particularly helpful for improving the perfomance of organic electronic devices employing such embodiments.

It may be provided that the substituents on one or more of the groups R¹ to R¹³ may themselves be substituted by substituents selected from the same group of substituents, for example may be alkyl substituted aryl or the like.

In a further embodiment, one of R¹ to R¹³ is independently selected from the group consisting of CN-substituted C₆ to C₄₂ aryl, CN-substituted C₃ to C₄₂ heteroaryl, and the remaining R¹ to R¹³ are independently selected from H or D, alternatively H.

In a further embodiment, R⁷ is selected from the group consisting of CN-substituted C₆ to C₄₂ aryl, CN-substituted C₃ to C₄₂ heteroaryl, and the remaining R¹ to R¹³ are independently selected from H or D, alternatively H.

In a further embodiment, R¹ to R¹³ are independently selected from the group consisting of substituted or unsubstituted C₆ to C₄₂ aryl and H, wherein the one or more substituent(s), if present, are independently selected from C₁ to C₁₈ alkyl

In a further embodiment, one of R¹ to R¹³ is CN-substituted C₆ to C₄₂ aryl, wherein further substituent(s), if present, are independently selected from the group consisting of C₁ to C₁₈ alkyl; and the remaining R¹ to R¹³ are independently selected from H or D, alternatively H.

In a further embodiment R⁷ is CN-substituted C₆ to C₄₂ aryl, wherein further substituent(s), if present, are independently selected from C₁ to C₁₈ alkyl; and the remaining R¹ to R⁶ and R⁸ to R¹³ are independently selected from H or D, alternatively H.

In a further embodiment at least one of R¹ to R¹³ is C₁₂ to C₁₈ aryl substituted with at least one CN-group and optionally further substituted with at least one C₁ to C₄ alkyl group.

In a further embodiment R⁷ is C₁₂ to C₁₈ aryl substituted with a CN-group and optionally further substituted with at least one C₁ to C₄ alkyl group and the remaining R¹ to R⁶ and R⁸ to R¹³ are independently selected from H or D, alternatively H.

In a further embodiment the compound (Ia) is a compound represented by one of the following structures A-1 to A-7

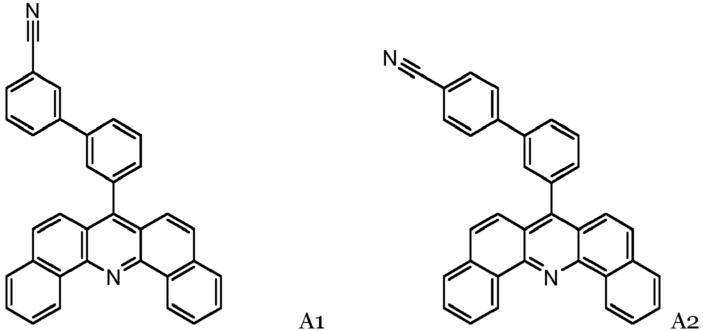

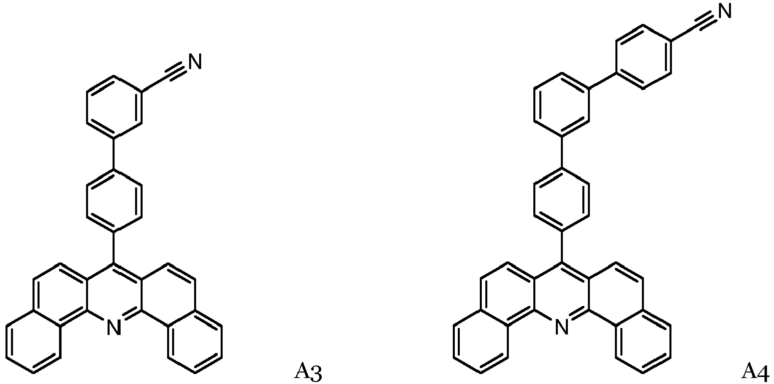

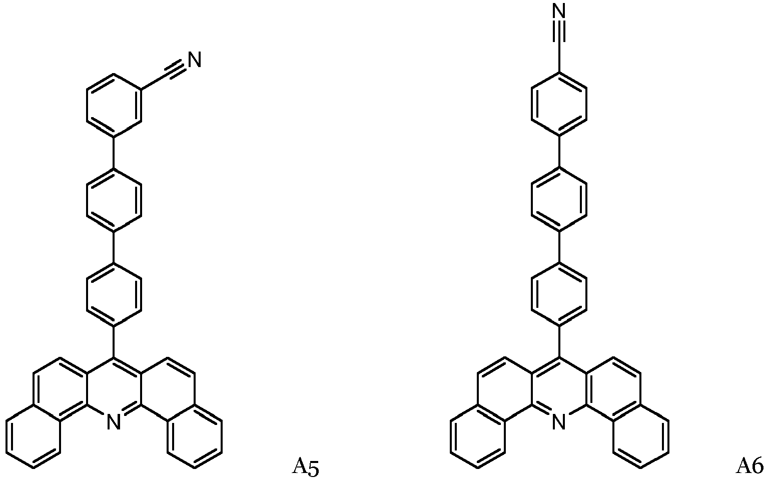

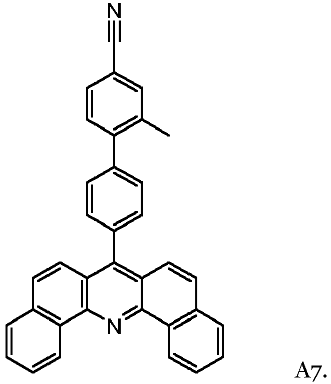

In a further embodiment the organic semiconducting material further comprises at least one electrical dopant.

In a further embodiment the electrical dopant is a n-dopant.

In a further embodiment the n-dopant is selected from the group consisting of metals, metal salts and metal complexes.

In a further embodiment the n-dopant comprises a metal cation selected from cations of alkali metals, alkaline earth metals, rare earth metals, transition metals, alternatively from cations of Li, Na, K, Rb, Cs, Mg, Ca, Sr, Ba, Mn, Fe, Co, Ni, and Zn.

In a further embodiment, the n-dopant comprises an anion or anionic ligand consisting of at least five covalently bound atoms.

The object is further achieved by an organic electornic device comprising a first electrode, a second electrode and a semiconducting layer arranged between the first electrode and the second electrode, wherein the semiconducting layer comprises the organic semiconducting material according to the invention.

In a further embodiment the semiconducting layer is an electron transport layer, an electron injection layer or an electron generating layer.

In a further embodiment the organic electronic device is an organic light emitting device, an organic photovoltaic device or an organic transistor.

In a further embodiment the organic light emitting device is an organic light emitting diode.

The object is further achieved by a display comprising at least one, alternatively at least two organic light emitting devices.

The display device may be an active matrix display device comprising at least two of the organic light emitting diodes.

Finally, the object is achieved by a compound (Ia) 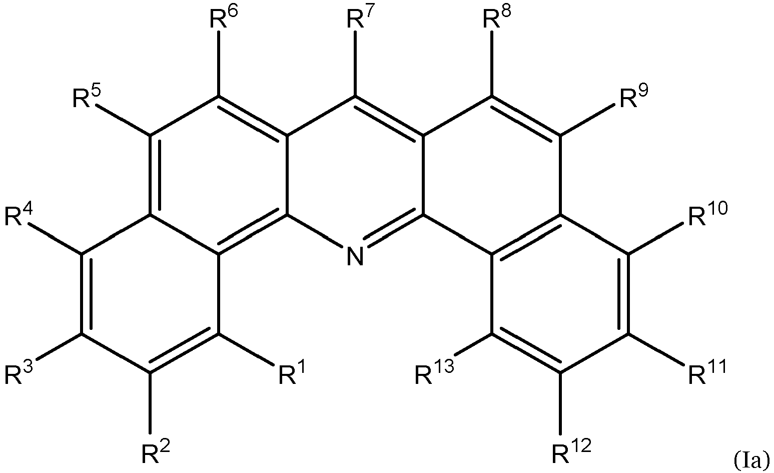
wherein R¹ to R¹³ are independently selected from the group consisting of H, D, F, Cl, Br, I, substituted or unsubstituted C₁ to C₁₈ alkyl, substituted or unsubstituted C₆ to C₄₂ aryl, substituted or unsubstituted C₃ to C₄₂ heteroaryl, wherein two or more groups selected from R¹ to R¹³ may be linked to form a non-aromatic ring;
wherein at least one of R¹ to R¹³ is a group substituted with at least one CN;
wherein the one or more substituent(s), if present in one of the groups R¹ to R¹³, are independently selected from the group consisting of D, F, Cl, Br, I, C₁ to C₁₈ alkyl, C₆ to C₃₆ aryl, C₆ to C₄₂ heteroaryl, R¹⁴R¹⁵P=O with R¹⁴ and R¹⁵ independently being C₁ to C₁₈ alkyl or C₆ to C₂₄ aryl, and CN
wherein compounds represented by the following formulas (1) to (22) are excluded

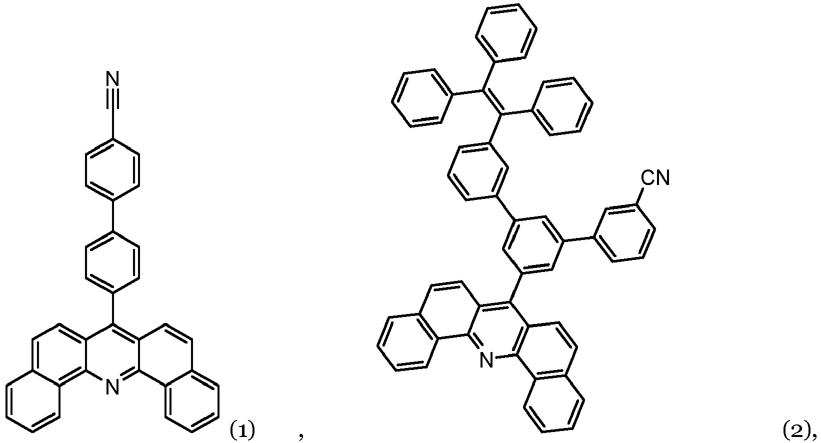

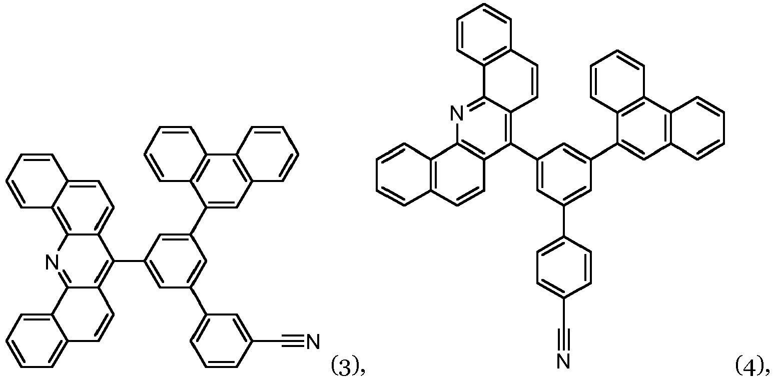

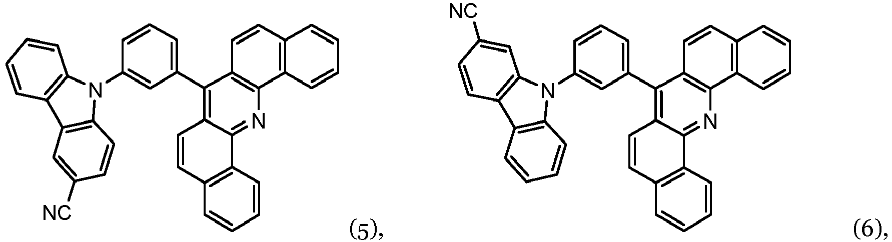

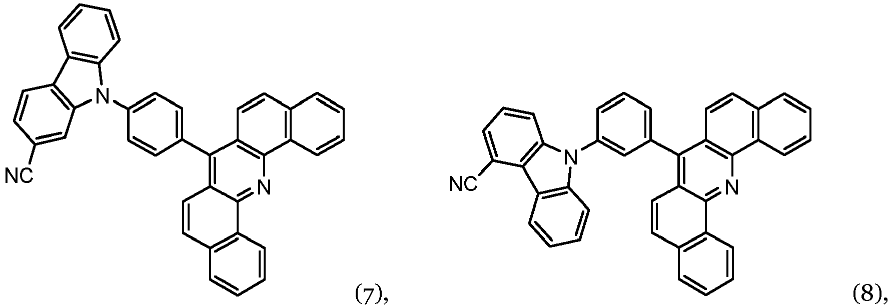

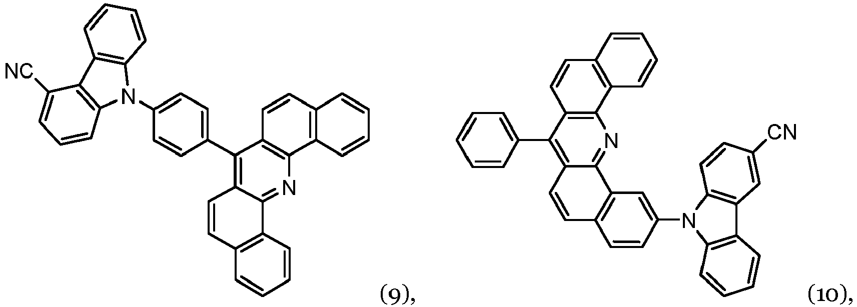

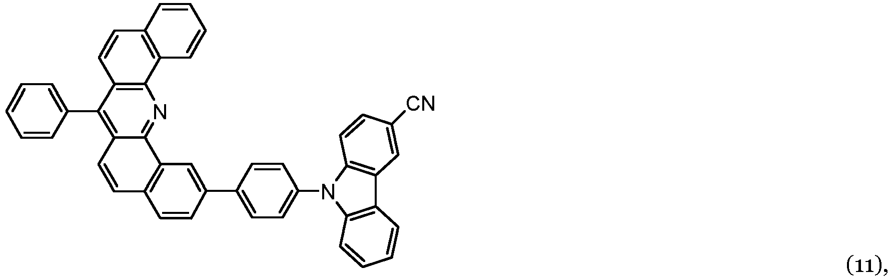

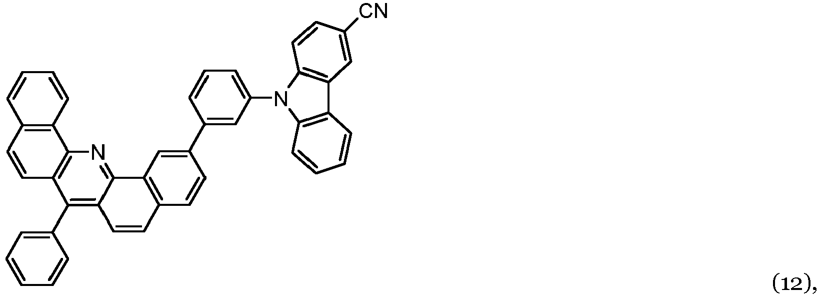

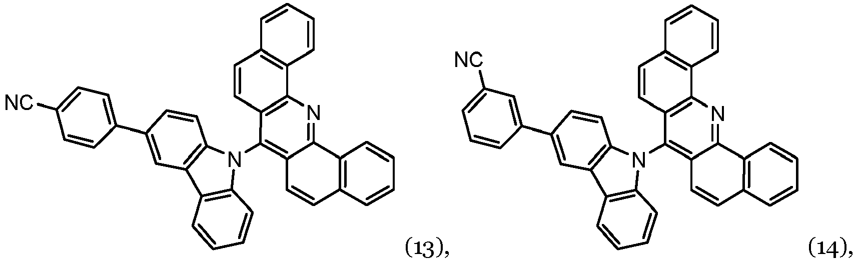

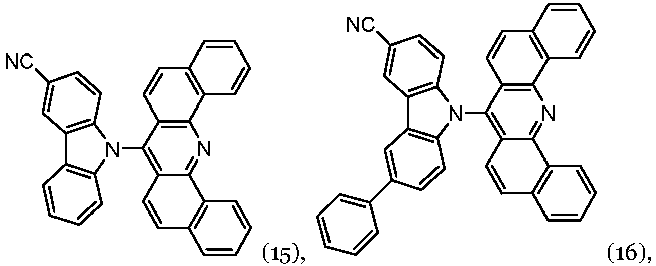

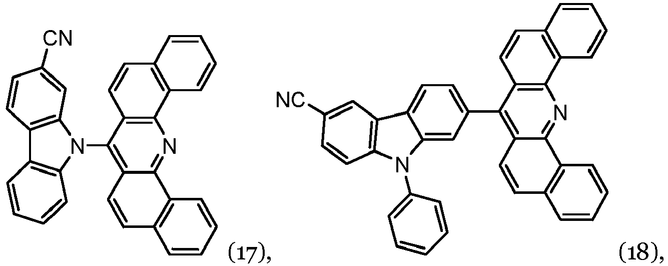

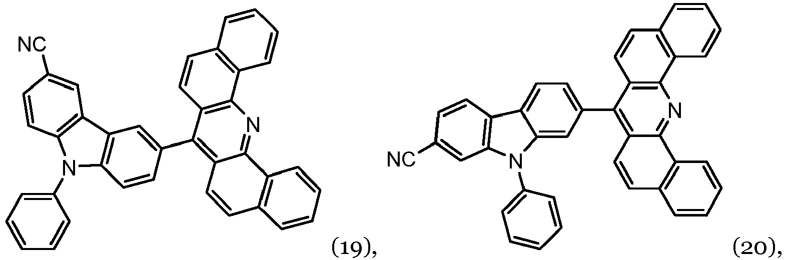

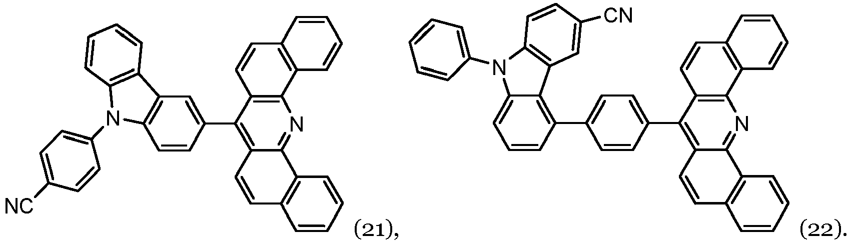

In a further embodiment the compound (I) has the following formula (Ia) 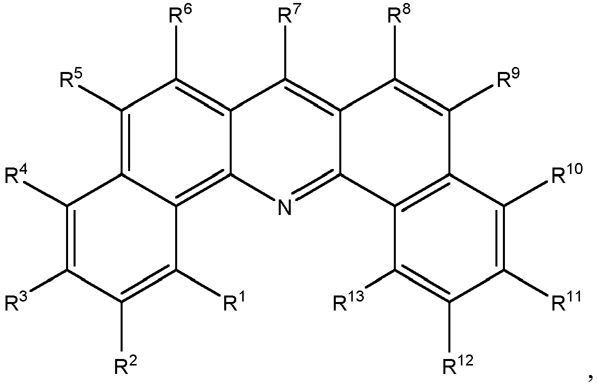
wherein R¹ to R¹¹ are independently selected from the group consisting of H, D, F, substituted or unsubstituted C₁ to C₁₈ alkyl, substituted or unsubstituted C₆ to C₄₂ aryl, substituted or unsubstituted C₃ to C₄₂ heteroaryl;
wherein at least one of R¹ to R¹³ is a group substituted with at least one CN;
wherein the one or more substituent(s), if present in one of the groups R¹ to R¹³, are independently selected from the group consisting of D, F, C1 to C₁₈ alkyl, C₆ to C₃₆ aryl, C₆ to C₄₂ heteroaryl, R¹⁴R¹⁵P=O with R¹⁴ and R¹⁵ independently being C₁ to C₁₈ alkyl or C₆ to C₂₄ aryl, and CN.

In a further embodiment one of R¹ to R¹³ is independently selected from the group consisting of CN-substituted C₆ to C₄₂ aryl, CN-substituted C₃ to C₄₂ heteroaryl, and the remaining R¹ to R¹³ are independently selected from H or D, alternatively H.

In a further embodiment R⁷ is selected from the group consisting of CN-substituted C₆ to C₄₂ aryl, CN-substituted C₃ to C₄₂ heteroaryl, and the remaining R¹ to R¹³ are independently selected from H or D, alternatively H.

In a further embodiment R¹ to R¹³ are independently selected from the group consisting of substituted or unsubstituted C₆ to C₄₂ aryl and H, wherein the one or more substituent(s), if present, are independently selected from C₁ to C₁₈ alkyl.

In a further embodiment one of R¹ to R¹³ is CN-substituted C₆ to C₄₂ aryl, wherein further substituent(s), if present, are independently selected from the group consisting of C₁ to C₁₈ alkyl; and the remaining R¹ to R¹³ are independently selected from H or D, alternatively H.

In a further embodiment R⁷ is CN-substituted C₆ to C₄₂ aryl, wherein further substituent(s), if present, are independently selected from C₁ to C₁₈ alkyl; and the remaining R¹ to R⁶ and R⁸ to R¹³ are independently selected from H or D, alternatively H.

In a further embodiment at least one of R¹ to R¹³ is C₁₂ to C₁₈ aryl substituted with at least one CN-group and optionally further substituted with at least one C₁ to C₄ alkyl group.

In a further embodiment R⁷ is C₁₂ to C₁₈ aryl substituted with a CN-group and optionally further substituted with at least one C₁ to C₄ alkyl group and the remaining R¹ to R⁶ and R⁸ to R¹³ are independently selected from H or D, alternatively H.

In a further embodiment the compound (Ia) is a compound represented by one of the following structures A-1 to A-7

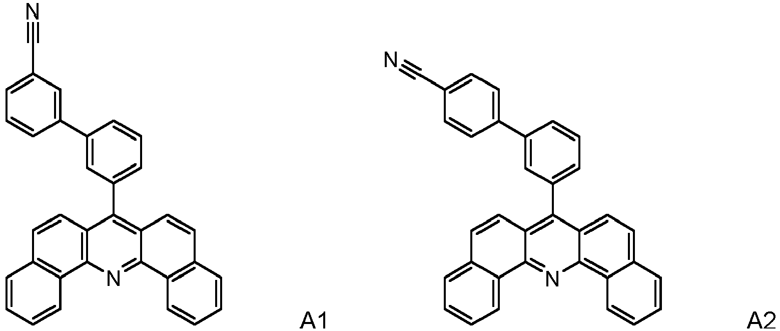

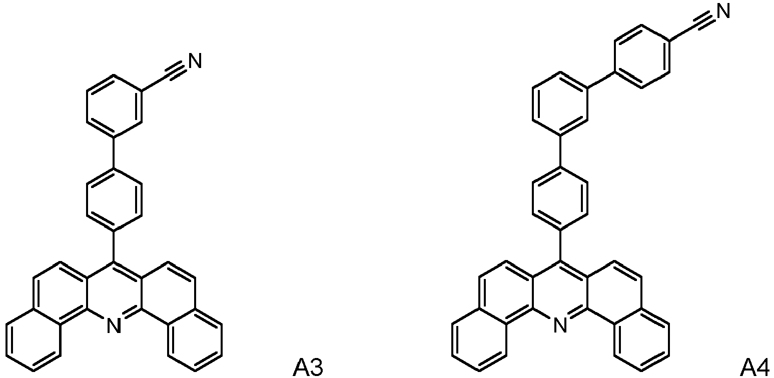

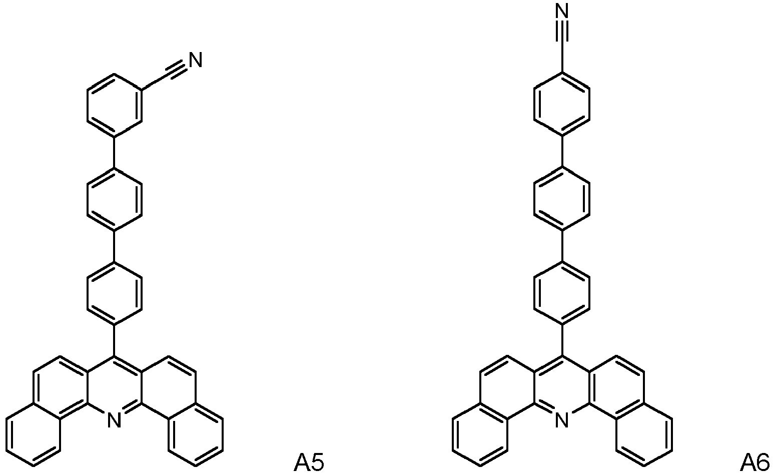

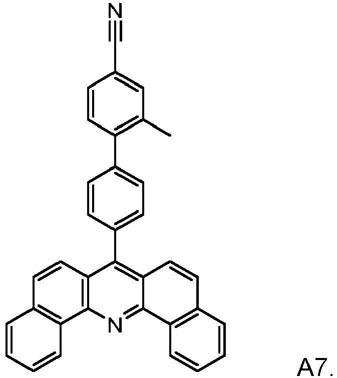

The structures of the example part of the present application are most preferred.

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### Anode electrode

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys.

### Hole injection layer

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise, or consist of, a p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations may be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

### Hole transport layer

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010.

Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from compounds with a triplet level above the triplet level of the phosphorescent emitter in the adjacent emission layer. Suitable compounds for the triplet control layer, in particular the triarylamine compounds, are described in EP 2 722 908 A1.

### Emission layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

The EML may be formed of a combination of host materials and emitter dopants. The EML may comprise a single host material or a plurality of host materials. The EML may comprise a single emitter dopant or a plurality of emitter dopants. Examples of the host materials are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

In case the EML comprises a plurality of host materials to form a host mixture the amount of each host material in the mixture of host materials may vary between 0.01 and 99.99 parts by weight.

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host or host mixture. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function. The hole blocking layer may be the inventive organic semiconducting layer comprising or consisting of the inventive compound (Ia) as defined above.

The HBL may be the layer (or one of several layers) comprising the compound.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazine derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The OLED according to the present invention may comprise an electron transport layer (ETL). In accordance with the invention, the electron transport layer may be the inventive organic semiconducting layer comprising the inventive compound (Ia). According to various embodiments the OLED may comprise an electron transport layer or an electron transport layer stack comprising at least a first electron transport layer and at least a second electron transport layer.

By suitably adjusting energy levels of particular layers of the ETL, the injection and transport of the electrons may be controlled, and the holes may be efficiently blocked. Thus, the OLED may have long lifetime.

The electron transport layer of the organic electronic device may comprise the compound (Ia) as defined above as the organic electron transport matrix (ETM) material. The electron transport layer may comprise, besides the compound (Ia), further ETM materials known in the art. Likewise, the electron transport layer may comprise as the only electron transport matrix material the compound (Ia). In case that the inventive organic electronic device comprises more than one electron transport layers, the compound (Ia) may be comprised in only one of the electron transport layers, in more than one of the electron transport layers or in all of the electron transport layers. In accordance with the invention, the electron transport layer may comprise, besides the ETM material, at least one additive as defined herein.

Further, the electron transport layer may comprise one or more n-type dopants. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another embodiment, the n-type dopant can be one selected from a group consisting of Cs, K, Rb, Mg, Na, Ca, Sr, Eu and Yb. In an embodiment the alkali metal compound may be 8-Hydroxyquinolinolato-lithium (LiQ), Lithium tetra(1H-pyrazol-1-yl)borate or Lithium 2-(diphenylphosphoryl)phenolate. Suitable compounds for the ETM (which may be used in addition to the inventive compound (Ia) as defined above) are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.

### Electron injection layer (EIL)

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL. The EIL may be the organic semiconducting layer comprising the compound (Ia).

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode electrode

The cathode electrode is formed on the EIL, if present. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed by a metal or metal alloy.

It is to be understood that the cathode electrode is not part of an electron injection layer or the electron transport layer.

### Charge generation layer

The charge generation layer (CGL) may comprise a p- type and an n-type layer. An interlayer may be arranged between the p-type layer and the n-type layer. The CGL may comprise the compound (Ia).

Typically, the charge generation layer is a pn junction joining an n-type charge generation layer (electron generating layer) and a hole generating layer. The n-side of the pn junction generates electrons and injects them into the layer which is adjacent in the direction to the anode. Analogously, the p-side of the p-n junction generates holes and injects them into the layer which is adjacent in the direction to the cathode.

Charge generating layers may be used in tandem devices, for example, in tandem OLEDs comprising, between two electrodes, two or more emission layers. In a tandem OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluoro-7,7,8,8-tetracyanoquinodimethane (F4-TCNQ), derivatives of tetracyanoquinodimethane, radialene derivatives, iodine, FeCl3, FeF3, and SbCl5. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB). The p-type charge generation layer may consist of CNHAT.

The n-type charge generating layer may be the layer comprising the compound (Ia). The n-type charge generation layer can be layer of a neat n-type dopant, for example of an electropositive metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline)aluminum, benzazole derivatives, and silole derivatives.

The hole generating layer is arranged in direct contact to the n-type charge generation layer.

### Organic light-emitting diode (OLED)

The organic electronic device according to the invention may be an organic light-emitting device.

According to one aspect of the present invention, there is provided an organic light-emitting diode (OLED) comprising: a substrate; an anode electrode formed on the substrate; a hole injection layer, a hole transport layer, an emission layer, an organic semiconducting layer comprising a compound (Ia) or consisting of a compound (Ia) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an organic semiconducting layer comprising a compound (Ia) or consisting of a compound (Ia) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an organic semiconducting layer comprising a compound (Ia) or consisting of a compound (Ia), an electron injection layer, and a cathode electrode.

According to various embodiments of the present invention, there may be provided OLEDs layers arranged between the above mentioned layers, on the substrate or on the top electrode.

According to one aspect, the OLED can comprise a layer structure of a substrate that is adjacently arranged to an anode electrode, the anode electrode is adjacently arranged to a first hole injection layer, the first hole injection layer is adjacently arranged to a first hole transport layer, the first hole transport layer is adjacently arranged to a first electron blocking layer, the first electron blocking layer is adjacently arranged to a first emission layer, the first emission layer is adjacently arranged to a first electron transport layer, the first electron transport layer is adjacently arranged to an n-type charge generation layer, the n-type charge generation layer is adjacently arranged to a hole generating layer, the hole generating layer is adjacently arranged to a second hole transport layer, the second hole transport layer is adjacently arranged to a second electron blocking layer, the second electron blocking layer is adjacently arranged to a second emission layer, between the second emission layer and the cathode electrode an optional electron transport layer and/or an optional injection layer are arranged.

The organic semiconducting layer according to the invention may be an emission layer, a hole blocking layer, an electron transport layer, a first electron transport layer, an n-type charge generation layer and/or a second electron transport layer.

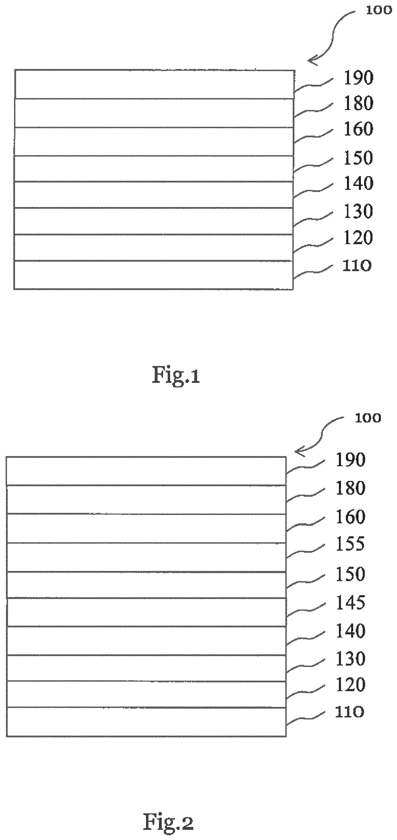

For example, the OLED (100) according to Fig. 2 may be formed by a process, wherein on a substrate (110), an anode (120), a hole injection layer (130), a hole transport layer (140), an electron blocking layer (145), an emission layer (150), a hole blocking layer (155), an electron transport layer (160), an electron injection layer (180) and the cathode electrode (190) are subsequently formed in that order.

### Organic electronic device

An organic electronic device according to the invention comprises an organic semiconducting layer comprising a compound (Ia) or consisting of a compound (Ia).

An organic electronic device according to one embodiment may include a substrate, an anode layer, an organic semiconducting layer comprising a compound (Ia) or consisting of a compound (Ia) and a cathode layer.

An organic electronic device according to one embodiment comprises at least one organic semiconducting layer comprising at least one compound (Ia) or consisting of a compound (Ia), at least one anode layer, at least one cathode layer and at least one emission layer, wherein the organic semiconducting layer is preferably arranged between the emission layer and the cathode layer.

An organic light-emitting diode (OLED) according to the invention may include an anode, a hole transport layer (HTL), an emission layer (EML), an electron transport layer (ETL) comprising at least one compound (Ia), and a cathode, which are sequentially stacked on a substrate. In this regard, the HTL, the EML, and the ETL are thin films formed from organic compounds.

An organic electronic device according to one embodiment can be a light emitting device, thin film transistor, a charge storage device, a display device or a photovoltaic cell, and preferably a light emitting device.

According to another aspect of the present invention, there is provided a method of manufacturing an organic electronic device, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing is selected from spin-coating, printing, casting; and/or
- slot-die coating.

According to various embodiments of the present invention, there is provided a method using:
- a first deposition source to release the compound (Ia) according to the invention,
   and
- a second deposition source to release a metal, a metal complex, an organo-metallic compound, a metal salt or an alkali or alkaline earth metal complex; alternatively an organic alkali or alkaline earth metal complex; alternatively 8-hydroxyquinolinolato lithium or alkali borate;
   the method comprising the steps of forming the organic semiconducting layer; whereby for an organic light-emitting diode (OLED):
   - the organic semiconducting layer is formed by releasing the compound (Ia) according to the invention from the first deposition source and a metal, a metal complex, an organo-metallic compound, a metal salt or an alkali or alkaline earth metal complex; alternatively an organic alkali or alkaline earth metal complex; alternatively 8-hydroxyquinolinolato lithium or alkali borate, from the second deposition source.

According to various embodiments of the present invention, the method may further include forming on the anode electrode, an emission layer and at least one layer selected from the group consisting of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein
- on a substrate a first anode electrode is formed,
- on the first anode electrode an emission layer is formed,
- on the emission layer an electron transport layer stack is formed, optionally a hole blocking layer is formed on the emission layer and an organic semiconducting layer is formed,
- and finally a cathode electrode is formed,
- optionally a hole injection layer, a hole transport layer, and a hole blocking layer, formed in that order between the first anode electrode and the emission layer,
- optionally an electron injection layer is formed between the organic semiconducting layer and the cathode electrode.

According to various embodiments of the present invention, the method may further comprise forming an electron injection layer on the organic semiconducting layer. However, according to various embodiments of the OLED of the present invention, the OLED may not comprise an electron injection layer.

According to various embodiments, the OLED may have the following layer structure, with the layers having the following order:
anode, hole injection layer, first hole transport layer, second hole transport layer, emission layer, optional hole blocking layer, organic semiconducting layer comprising a compound (Ia)
according to the invention, optional electron injection layer, and cathode, or
anode, hole injection layer, first hole transport layer, second hole transport layer,
organic semiconducting layer comprising a compound (Ia) according to the invention,
optional hole blocking layer, first electron transport layer, optional electron injection layer, and cathode, or
anode, hole injection layer, first hole transport layer, second hole transport layer,
emission layer, organic semiconducting layer comprising a compound (Ia) according to
the invention, first electron transport layer, optional electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

In one embodiment, the organic electronic device according to the invention comprising an organic semiconducting layer comprising a compound (Ia) or consisting of a compound (Ia) may further comprise a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb): 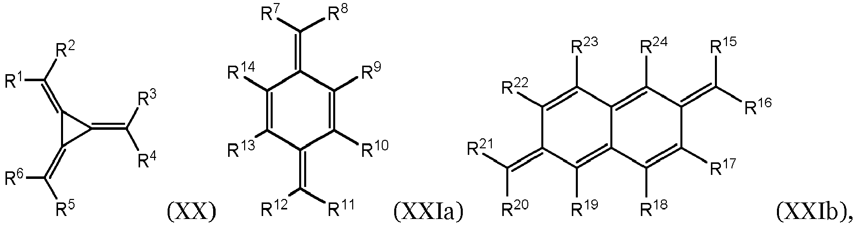 wherein R¹, R², R³, R⁴, R⁵, R⁶, R⁷, R⁸, R¹¹, R¹², R¹⁵, R¹⁶, R²⁰, R²¹ are independently selected from above mentioned electron withdrawing groups and R⁹, R¹⁰, R¹³, R¹⁴, R¹⁷, R¹⁸, R¹⁹, R²², R²³ and R²⁴ are independently selected from H, halogen and above mentioned electron withdrawing groups.

Hereinafter, the embodiments are illustrated in more detail with reference to examples. However, the present disclosure is not limited to the following examples. Reference will now be made in detail to the exemplary aspects.

### Details and definitions of the invention

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, C₃-alkyl may be selected from n-propyl and iso-propyl. Likewise, C₄-alkyl encompasses n-butyl, sec-butyl and t-butyl. Likewise, C₆-alkyl encompasses n-hexyl and cyclo-hexyl.

The subscribed number n in Cₙ relates to the total number of carbon atoms in the respective alkyl, arylene, heteroarylene or aryl group.

The term "aryl" or "arylene" as used herein shall encompass phenyl (C₆-aryl), fused aromatics, such as naphthalene, anthracene, phenanthracene, tetracene etc. Further encompassed are biphenyl and oligo- or polyphenyls, such as terphenyl etc.. Further encompassed shall be any further aromatic hydrocarbon substituents, such as fluorenyl etc. "Arylene" respectively "heteroarylene", referes to groups to which two further moieties are attached. In the present specification the term "aryl group" or "arylene group" may refer to a group comprising at least one hydrocarbon aromatic moiety, and all the elements of the hydrocarbon aromatic moiety may have p-orbitals which form conjugation, for example a phenyl group, a naphtyl group, an anthracenyl group, a phenanthrenyl group, a pyrenyl group, a fluorenyl group and the like. The aryl or arylene group may include a monocyclic or fused ring polycyclic (i.e., rings sharing adjacent pairs of carbon atoms) functional group.

The term "heteroaryl" as used herein refers to aryl groups in which at least one carbon atom is substituted with a heteroatom, preferably selected from N, O, S, B or Si.

The subscripted number n in Cₙ-heteroaryl merely refers to the number of carbon atoms excluding the number of heteroatoms. In this context, it is clear that a C₃ heteroarylene group is an aromatic compound comprising three carbon atoms, such as pyrazol, imidazole, oxazole, thiazole and the like.

The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms. Preferably a heteroarylene ring may comprise at least 1 to 3 heteroatoms individually selected from N, S and/or O.

The term "heteroaryl" as used herewith shall encompass pyridine, quinoline, quinazoline, pyridine, triazine, benzimidazole, benzothiazole, benzo[4,5]thieno[3,2-d]pyrimidine, carbazole, xanthene, phenoxazine, benzoacridine, dibenzoacridine and the like.

In the present specification, the single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

With respect to the inventive organic semiconducting layer as well as with respect to the inventive compound, the compounds mentioned in the experimental part are most preferred.

The inventive organic electronic device may be an organic electroluminescent device (OLED) an organic photovoltaic device (OPV), a lighting device, or an organic field-effect transistor (OFET). A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.

According to another aspect, the organic electroluminescent device according to the present invention may comprise more than one emission layer, preferably two or three emission layers. An OLED comprising more than one emission layer is also described as a tandem OLED or stacked OLED.

The organic electroluminescent device (OLED) may be a bottom- or top-emission device.

Another aspect is directed to a device comprising at least one organic electroluminescent device (OLED).

A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural Formula.

The terms "OLED" and "organic light-emitting diode" are simultaneously used and have the same meaning. The term "organic electroluminescent device" as used herein may comprise both organic light emitting diodes as well as organic light emitting transistors (OLETs).

As used herein, "weight percent", "wt.-%", "percent by weight", "% by weight", parts by weight and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of", "does not contain", "does not comprise" does not exclude impurities. Impurities have no technical effect with respect to the object achieved by the present invention.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

Preferably, the organic semiconducting layer comprising the compound (Ia) is essentially non-emissive or non-emitting.

The operating voltage, also named U, is measured in Volt (V) at 10 milliAmpere per square centimeter (mA/cm2).

The candela per Ampere efficiency, also named cd/A efficiency is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device" and "organic light-emitting diode" are simultaneously used and have the same meaning.

The term "life-span" and "lifetime" are simultaneously used and have the same meaning.

The anode electrode and cathode electrode may be described as anode electrode / cathode electrode or anode electrode / cathode electrode or anode electrode layer / cathode electrode layer.

Room temperature, also named ambient temperature, is 23° C.

### BRIEF DESCRIPTION OF THE DRAWINGS

These and/or other aspects and advantages of the present invention will become apparent and more readily appreciated from the following description of the exemplary embodiments, taken in conjunction with the accompanying drawings, of which:
FIG. 1 is a schematic sectional view of an organic light-emitting diode (OLED), according to an exemplary embodiment of the present invention;
FIG. 2 is a schematic sectional view of an OLED, according to an exemplary embodiment of the present invention.
FIG. 3 is a schematic sectional view of a tandem OLED comprising a charge generation layer, according to an exemplary embodiment of the present invention.

### DETAILED DESCRIPTION

Reference will now be made in detail to the exemplary embodiments of the present invention, examples of which are illustrated in the accompanying drawings, wherein like reference numerals refer to the like elements throughout. The exemplary embodiments are described below, in order to explain the aspects of the present invention, by referring to the figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an electron transport layer (ETL) 160. The electron transport layer (ETL) 160 is formed on the EML 150. Onto the electron transport layer (ETL) 160, an electron injection layer (EIL) 180 is disposed. The cathode 190 is disposed directly onto the electron injection layer (EIL) 180.

Instead of a single electron transport layer 160, optionally an electron transport layer stack (ETL) can be used.

Fig. 2 is a schematic sectional view of an OLED 100, according to another exemplary embodiment of the present invention. Fig. 2 differs from Fig. 1 in that the OLED 100 of Fig. 2 comprises an electron blocking layer (EBL) 145 and a hole blocking layer (HBL) 155.

Referring to Fig. 2, the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode electrode 190.

Preferably, the organic semiconducting layer comprising a compound (Ia) or consisting of a compound (Ia) may be an EML, an HBL or an ETL.

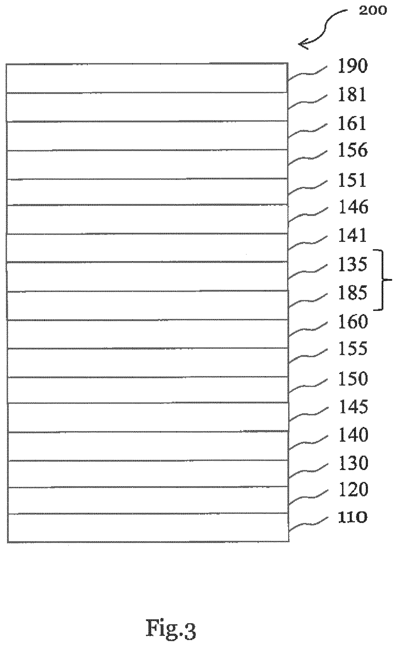

Fig. 3 is a schematic sectional view of a tandem OLED 200, according to another exemplary embodiment of the present invention. Fig. 3 differs from Fig. 2 in that the OLED 200 of Fig. 3 further comprises a charge generation layer (CGL) and a second emission layer (151).

Referring to Fig. 3, the OLED 200 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (EBL) 156, a second electron transport layer (ETL) 161, a second electron injection layer (EIL) 181 and a cathode 190.

Preferably, the organic semiconducting layer comprising a compound (Ia) or consisting of a compound (Ia) may be the first EML, first HBL, first ETL, n-type CGL and/or second EML, second HBL, second ETL.

While not shown in Fig. 1, Fig. 2 and Fig. 3, a sealing layer may further be formed on the cathode electrodes 190, in order to seal the OLEDs 100 and 200. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Experimental Part

The invention is furthermore illustrated by the following examples which are illustrative only and non- binding.

Preparation of inventive compounds:

### SYNTHESIS OF PRECURSORS

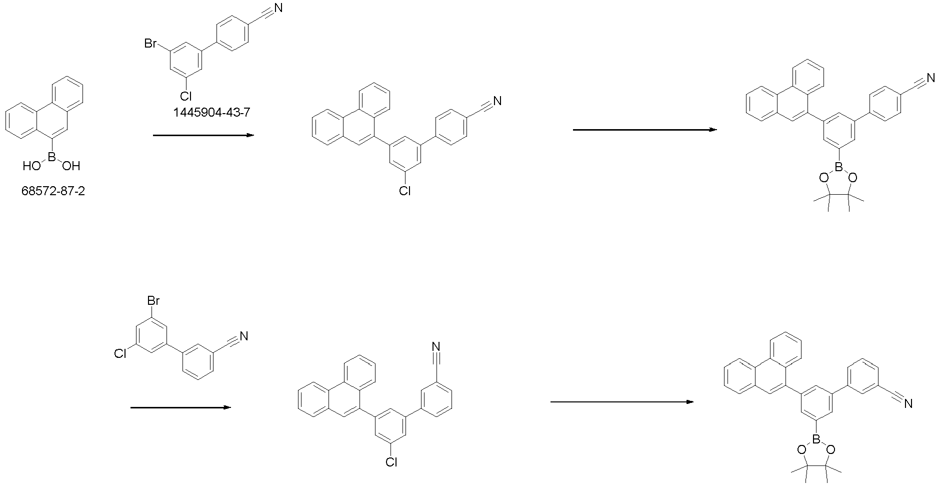

### 3'-bromo-5'-chloro-[1,1'-biphenyl]-3-carbonitrile

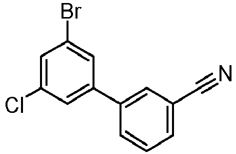

3'-bromo-5'-chloro-[1,1'-biphenyl]-3-carbonitrile was synthesized following the procedure described in the literature for the synthesis of 3'-bromo-5'-chloro-[1,1'-biphenyl]-4-carbonitrile (Journal of Medicinal Chemistry, 56(13), 5473-5494; 2013)

### 3'-chloro-5'-(phenanthren-9-yl)-[1,1'-biphenyl]-4-carbonitrile

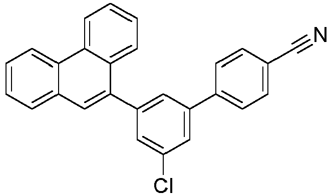

A flask was flushed with nitrogen and charged with phenanthren-9-ylboronic acid (9.7 g, 43.8 mmol), 3'-bromo-5'-chloro-[1,1'-biphenyl]-4-carbonitrile (12.8 g, 43.8 mmol), tetrakis(triphenylphosphin)palladium(o) (1.0 g, 0.9 mmol), and potassium carbonate (12.0 g, 87.5 mmol). A mixture of deaerated toluene / THF / water (1:1:0.5, 174 mL) was added and the reaction mixture was heated to 90 °C under a nitrogen atmosphere over 23 hours. After cooling down to room temperature, the solvent was removed under reduced pressure. The crude product was then dissolved in chloroform (600 mL) and the organic phase was washed with water (4 × 100 mL). After drying over MgSO₄, the organic phase was filtered through a silicagel pad. The filtrate was concentrated under reduced pressure and n-hexane (250 mL) was added to the resulting oil. The resulting suspension was stirred at room temperature overnight. The precipitate was collected by suction filtration and washed with n-hexane. Crude product was used directly in the next step.

### SYNTHESIS OF FINAL COMPOUNDS

### 3'-(dibenzo[c,h]acridin-7-yl)-[1,1'-biphenyl]-3-carbonitrile A1

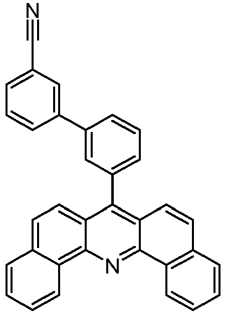

A flask was flushed with argon and charged with 7-(3-bromophenyl)dibenzo[c,h]acridine (15.0 g, 34.0 mmol), (3-cyanophenyl)boronic acid (6.1 g, 41.4 mmol), potassium carbonate (14.3 g, 103.6 mmol) in deaerated water (45 mL) and deaerated glyme (175 mL). Tetrakis(triphenylphosphin)palladium(o) (798 mg, 0.69 mmol) was added and the reaction mixture was heated to 95 °C under an argon atmosphere overnight. After cooling down to room temperature, the resulting precipitate was isolated by suction filtration and washed with glyme (2 × 5 mL), water (500 mL), glyme (5 ml) again and n-hexane (2 × 10 mL). The crude product was then dissolved in dichloromethane (500 mL) and filtered through a pad of silica. After rinsing with additional dichloromethane (400 mL), the filtrate was concentrated under reduced pressure. The resulting precipitate was isolated by suction filtration and washed with n-hexane. Finally, it was dissolved in dichloromethane (300 mL). Toluene (100 mL) was added and the dichloromethane was removed under reduced pressure. The resulting precipitate was recrystallized in dimethylformamide (70 mL) to yield 8.8 g (55%) of a colourless solid after drying. Final purification was achieved by sublimation. HPLC/ESI-MS: m/z = 457 ([M+H]+).

### 3'-(dibenzo[c,h]acridin-7-yl)-[1,1'-biphenyl]-4-carbonitrile A2

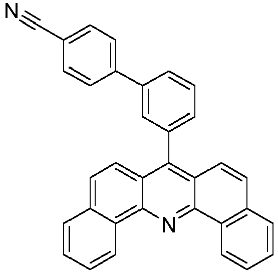

A flask was flushed with argon and charged with 7-(3-bromophenyl)dibenzo[c,h]acridine (20.0 g, 46.0 mmol), (4-cyanophenyl)boronic acid (8.1 g, 55.3 mmol) and tetrakis(triphenylphosphin)palladium(o) (1.1 g, 0.9 mmol) A deaerated aqueous solution of potassium carbonate (19.1 g, 138.0 mmol), water (70 mL) and deaerated glyme (230 mL) were added and the reaction mixture was heated to 95 °C under an argon atmosphere overnight. After cooling down to room temperature, the phases were separated and glyme was removed under reduced pressure. The crude product was then dissolved in dichloromethane and filtered through a pad of silica. After rinsing with additional dichloromethane, the filtrate was concentrated under reduced pressure, n-hexane was added and the solution was stirred over 1 hour. The resulting precipitate was isolated by suction filtration and washed with n-hexane. The crude product was further purified by recrystallization from toluene to yield 19.2 g (91%) of a colourless solid after drying. Final purification was achieved by sublimation. HPLC/ESI-MS: m/z = 457 ([M+H]+).

### 4'-(dibenzo[c,h]acridin-7-yl)-[1,1'-biphenyl]-3-carbonitrile A3

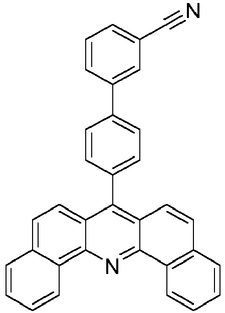

A flask was flushed with nitrogen and charged with 7-(4-bromophenyl)dibenzo[c,h]acridine (10.0 g, 23.0 mmol), (3-cyanophenyl)boronic acid (4.1 g, 27.6 mmol) and potassium carbonate (9.5 g, 69.0 mmol). A mixture of deaerated glyme / water - 6.5:1 (265 mL) and tetrakis(triphenylphosphin)palladium(0) (532 mg, 0.5 mmol) were added and the reaction mixture was heated to 100 °C under a nitrogen atmosphere overnight. After cooling down to room temperature, the resulting precipitate was isolated by suction filtration and washed with toluene (50 mL), water (40 mL) and methanol (60 mL). The crude product was then dissolved in hot chlorobenzene (500 mL), filtered through a pad of silica and rinsed with additional hot chlorobenzene (600 mL). After cooling down to room temperature overnight, the resulting precipitate was isolated by suction filtration to yield 6.2 g (57%) of a bright yellow solid after drying. Final purification was achieved by sublimation. HPLC/ESI-MS: m/z = 457.1 ([M+H]⁺).**4**"-**(dibenzo[c,h]acridin-7-yl)-[1,1':3',1"-terphenyl]-4-carbonitrile A4**

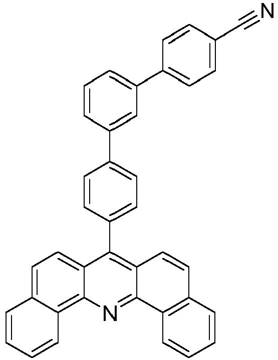

A suspension of 7-(4-bromophenyl)dibenzo[c,h]acridine (10.0 g, 23.0 mmol), 3'-(4,4,5,5-tetramethyl-1,3,2-dioxaborolan-2-yl)-[1,1'-biphenyl]-4-carbonitrile (8.4 g, 27.6 mmol) and [1,1'-bis(diphenylphosphino)ferrocene]dichloropalladium(II) (505 mg, 0.69 mmol) in toluene / THF / water - 4:1:1 (140 mL) was degassed with nitrogen. Potassium carbonate (6.36 g, 46.0 mmol) was added and the reaction mixture was heated to 70 °C under a nitrogen atmosphere overnight. After cooling down to room temperature, the resulting precipitate was isolated by suction filtration and washed with water (50 mL), methanol (50 mL), toluene (2 × 50 mL) and additional methanol (50 mL). The crude product was then dissolved in hot chloroform and filtered through a pad of florisil. After rinsing with additional hot chloroform (250 mL), the filtrate was concentrated under reduced pressure to a volume of 200 mL and n-hexane (200 mL) was added. The resulting precipitate was collected by suction filtration and washed with n-hexane (50 mL). The crude product was further purified by recrystallization from chlorobenzene to yield 8.7 g (71%) of a light yellow solid after drying. Final purification was achieved by sublimation. HPLC/ESI-MS: m/z = 533.1 ([M+H]⁺).

### 4"-(dibenzo[c,h]acridin-7-yl)-[1,1':4',1"-terphenyl]-3-carbonitrile A5

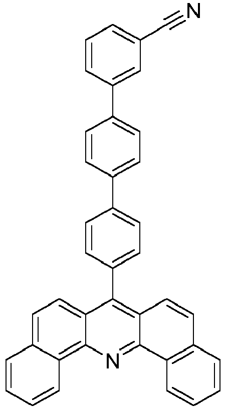

A suspension of 7-(4-(4,4,5,5-tetramethyl-1,3,2-dioxaborolan-2-yl)phenyl)dibenzo[c,h]acridine (5.0 g, 10.4 mmol), 4'-bromo-[1,1'-biphenyl]-3-carbonitrile (3.2 g, 12.5 mmol) and [1,1'-Bis(diphenylphosphino)ferrocene]dichloropalladium(II) (228 mg, 0.3 mmol) in toluene / THF / water - 4:1:1 (140 mL) was degassed with nitrogen. Potassium carbonate (6.4 g, 46.0 mmol) was added and the reaction mixture was heated to 70 °C under a nitrogen atmosphere overnight. Additional [1,1'-Bis(diphenylphosphino)ferrocene]dichloropalladium(II) (114 mg, 0.15 mmol) and toluene (50 mL) was added and the reaction mixture was heated to 80 °C under a nitrogen atmosphere overnight. After cooling down to room temperature, the resulting precipitate was isolated by suction filtration and washed with toluene, water and methanol. The crude product was further purified by Soxhlet extraction with chlorobenzene overnight. After cooling down to room temperature, the resulting precipitate was isolated by suction filtration and washed with methanol to yield 2.6 g (47%) of a beig solid after drying. Final purification was achieved by sublimation. HPLC/ESI-MS: 99.94%, m/z = 533.1 ([M+H]⁺).

### 4"-(dibenzo[c,h]acridin-7-yl)-[1,1':4',1"-terphenyl]-4-carbonitrile A6

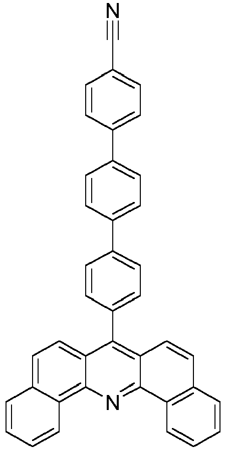

A flask was flushed with nitrogen and charged with 7-(4-bromophenyl)dibenzo[c,h]acridine (10.0 g, 23.0 mmol), 4'-(4,4,5,5-tetramethyl-1,3,2-dioxaborolan-2-yl)-[1,1'-biphenyl]-4-carbonitrile (8.4 g, 27.6 mmol) and [1,1'-Bis(diphenylphosphino)ferrocene]dichloropalladium(II) (505 mg, 0.7 mmol). A mixture of deaerated Toluene/THF 7:3 (230 mL) and a deaerated solution of potassium carbonate (6.4 g, 46.0 mmol) in water (23 mL) was added and the reaction mixture was heated to 80 °C under a nitrogen atmosphere overnight. After cooling down to room temperature, the resulting precipitate was isolated by suction filtration and washed with water and methanol. The crude product was further purified by soxhlet extraction with chlorobenzene over 48 h. After cooling down to room temperature, the resulting precipitate was isolated by suction filtration, washed with methanol and further purified by trituration in chlorobenzene to yield 7.0g (57%) of a slightly grey solid. Final purification was achieved by sublimation. HPLC/ESI-MS: m/z = 533.2 ([M+H]⁺).

### 4'-(dibenzo[c,h]acridin-7-yl)-2-methyl-[1,1'-biphenyl]-4-carbonitrile A7

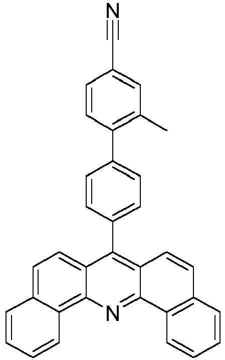

A flask was flushed with nitrogen and charged with 7-(4-(4,4,5,5-tetramethyl-1,3,2-dioxaborolan-2-yl)phenyl)dibenzo[c,h]acridine (10.0 g, 20.8 mmol) and 4-bromo-3-methylbenzonitrile (4.5 g, 22.9 mmol). A deaerated solution of potassium carbonate (5.74 g, 41.5 mmol) in water (21 mL), a mixture of deaerated (toluene / THF - 3:1) (170 mL) and [1,1'-bis(diphenylphosphino)ferrocene]dichloropalladium(II) (304 mg, 0.4 mmol) were added and the reaction mixture was heated to 80 °C under a nitrogen atmosphere over 96 h. After cooling down to room temperature, the resulting precipitate was isolated by suction filtration and washed with water (700 mL) and methanol (20 mL). The crude product was then dissolved in hot chlorobenzene (800 mL) and filtered through a pad of silica. After rinsing with additional hot chlorobenzene (500 mL), the filtrate was concentrated under reduced pressure to a volume of 200 mL. After cooling down to room temperature, the resulting precipitate was isolated by suction filtration and washed with n-hexane (10 mL) to yield 7.9 g (81%) of a yellow solid after drying. Final purification was achieved by sublimation. HPLC/ESI-MS: m/z = 471.2 ([M+H]⁺).

### Device examples

### Auxiliary materials

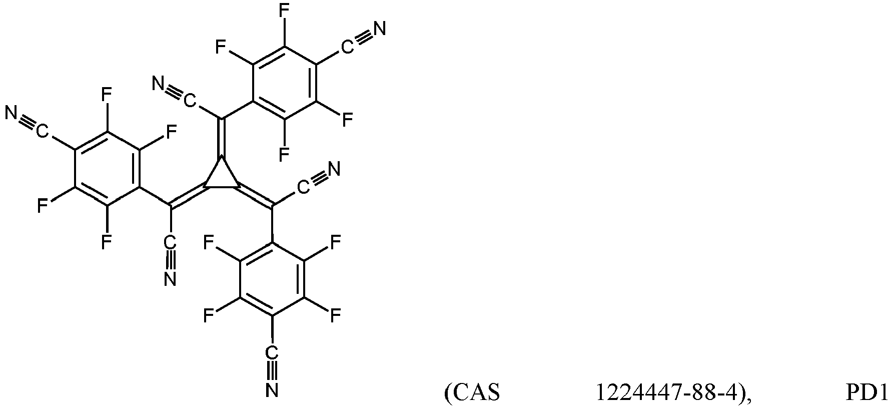

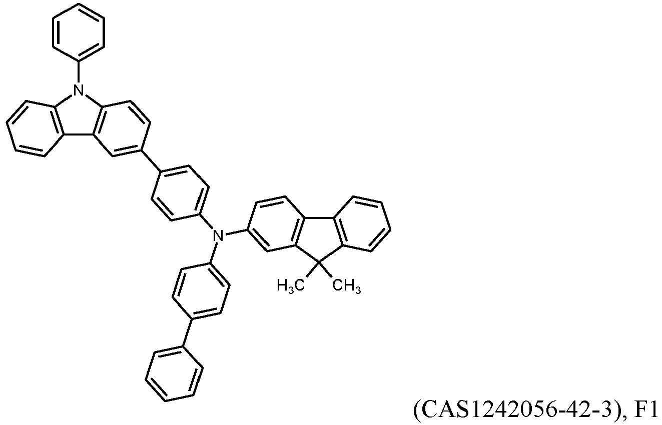

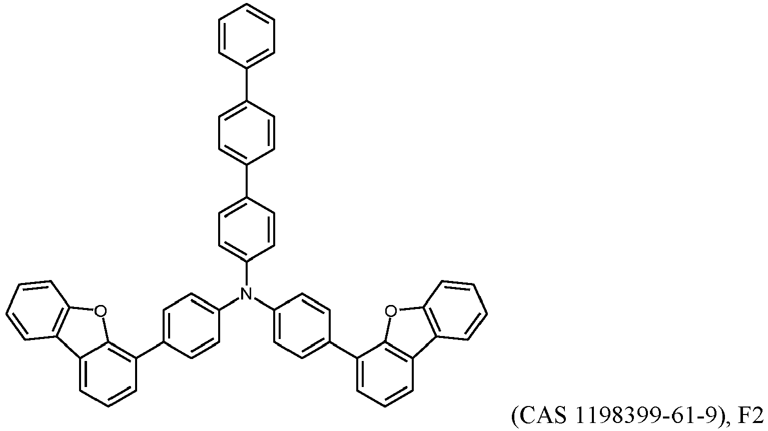

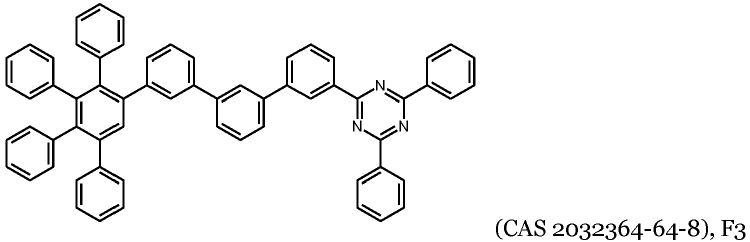

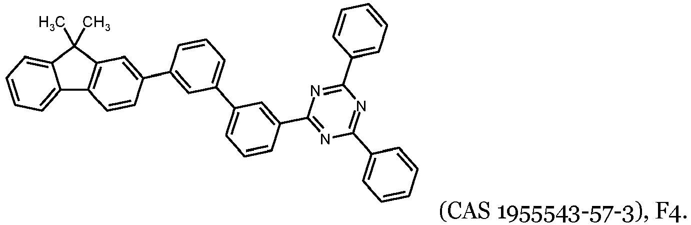

H09 is an emitter host and BD200 is a fluorescent emitter, both supplied by SFC, Korea.

LiQ is lithum 8-hydroxyquinolinolate.

Comparative state-of-art compounds

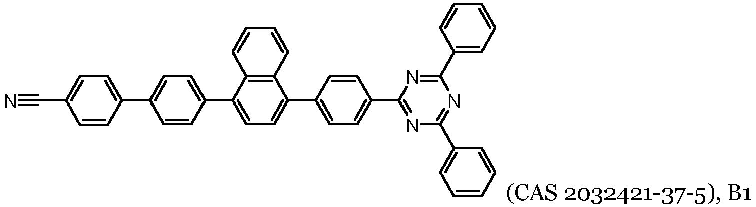

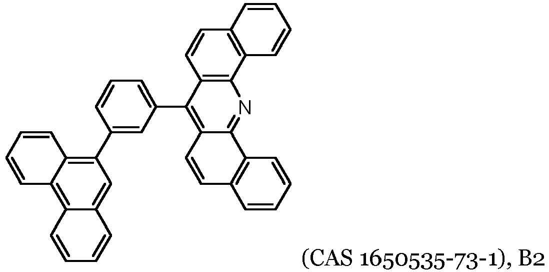

### Experimental blue OLED I

On a 100 nm thick silver anode vacuum deposited on a glass substrate, following layers were vacuum deposited in the given order: 10 nm thick HIL consisting of F1 and PD1 in weight ratio 92:8; 118 nm thick HTL consisting of neat F1, 5 nm thick EBL consisting of neat F2, 20 nm thick EML consisting of H09 and BD200 in weight ratio 97:3, 20 nm thick HBL consisting of F3, 31 nm thick ETL consisting of the inventive or comparative electron transport matrix compound and LiQ in weight ratio 50:50; 2 nm thick EIL consisting of neat Yb, 13 nm thick cathode consisting of Ag and Mg in weight ratio 90:10, and 75 nm thick cap layer consisting of neat F1. The obtained results are shown in Table 1.

**Table 1**

| compound | Tg [°C] | Voltage [V] | Current efficiency [cd/A] | lifetime [h] |
|---|---|---|---|---|
| A1 | 97 | 3.54 | 7.47 | 62 |
| A2 | 109 | 3.57 | 7.62 | 53 |
| A3 | 103 | 3.53 | 7.21 | 49 |
| A4 | 124 | 3.49 | 7.14 | 32 |
| A5 | 120 | 3.58 | 7.41 | 55 |
| A6 | | 3.63 | 7.31 | 67 |
| A7 | | 3.52 | 7.80 | 64 |
| B1 | 117 | 3.59 | 7.21 | 88 |
| B2 | 123 | 3.51 | 8.62 | 32 |

### Experimental blue OLED II

In the experimental blue OLED I, the auxiliary HBL compound F3 has been replaced with compound F4. The obtained results are shown in Table 2.

**Table 2**

| compound | Voltage [V] | Current efficiency [cd/A] | lifetime [h] |
|---|---|---|---|
| A1 | 3.63 | 7.0 | 94 |
| A2 | 3.61 | 7.1 | 78 |
| A3 | 3.60 | 6.9 | 90 |
| A4 | 3.57 | 6.8 | 106 |
| A5 | 3.67 | 6.7 | 92 |
| A6 | 3.72 | 6.7 | 99 |
| A7 | 3.63 | 7.1 | 100 |
| B1 | 3.70 | 6.4 | 141 |

The features disclosed in the foregoing description and in the dependent claims may, both separately and in any combination thereof, be material for realizing the aspects of the disclosure made in the independent claims, in diverse forms thereof.

### Experimental blue OLED II

In the experimental blue OLED I, the auxiliary HBL compound F3 has been replaced with compound F4. The obtained results are shown in Table 2.

**Table 2**

| compound | Voltage [V] | Current efficiency [cd/A] | lifetime [h] |
|---|---|---|---|
| A1 | 3.63 | 7.0 | 94 |
| A2 | 3.61. | 7.1 | 78 |
| A3 | 3.60 | 6.9 | 90 |
| A4 | 3.57 | 6.8 | 106 |
| A5 | 3.67 | 6.7 | 92 |
| A6 | 3.72 | 6.7 | 99 |
| A7 | 3.63 | 7.1 | 100 |
| B1 | 3.70 | 6.4 | 141 |

## Claims

1. Organic semiconducting material comprising a compound represented by the following formula (Ia) 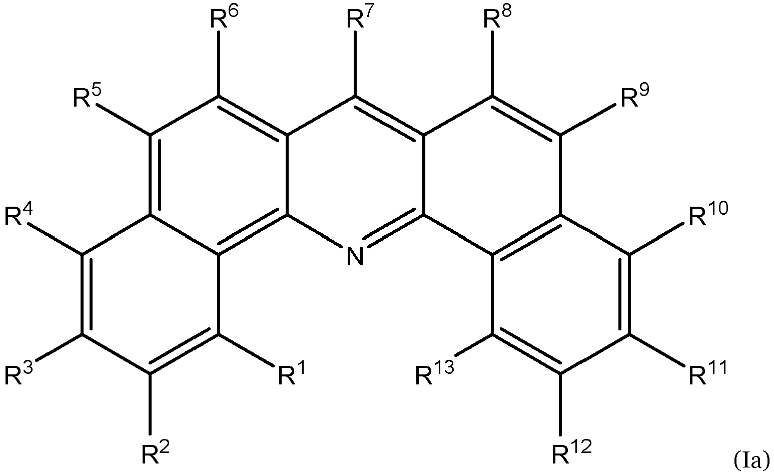
wherein R¹ to R¹³ are independently selected from the group consisting of H, D, F, Cl, Br, I, substituted or unsubstituted C₁ to C₁₈ alkyl, substituted or unsubstituted C₆ to C₄₂ aryl, substituted or unsubstituted C₃ to C₄₂ heteroaryl, wherein two or more groups selected from R¹ to R¹³ may be linked to form a non-aromatic ring;
wherein at least one of R¹ to R¹³ is a group substituted with at least one CN;
wherein the one or more substituent(s), if present in one of the groups R¹ to R¹³, are independently selected from the group consisting of D, F, Cl, Br, I, C₁ to C₁₈ alkyl, C₆ to C₃₆ aryl, C₆ to C₄₂ heteroaryl, R¹⁴R¹⁵P=O with R¹⁴ and R¹⁵ independently being C₁ to C₁₈ alkyl or C₆ to C₂₄ aryl, and CN,
wherein the compounds represented by the following formulas (1) to (5) are excluded

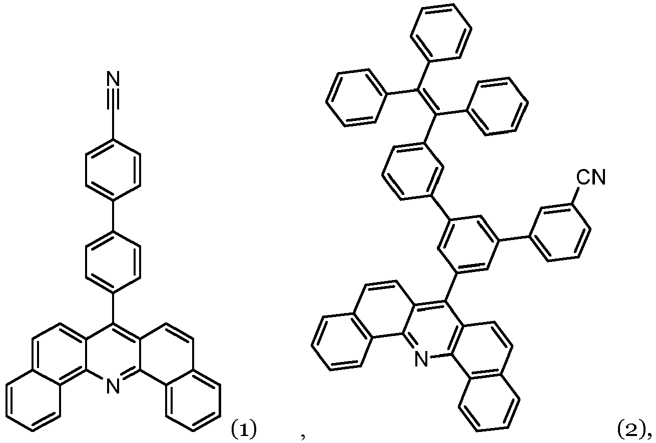

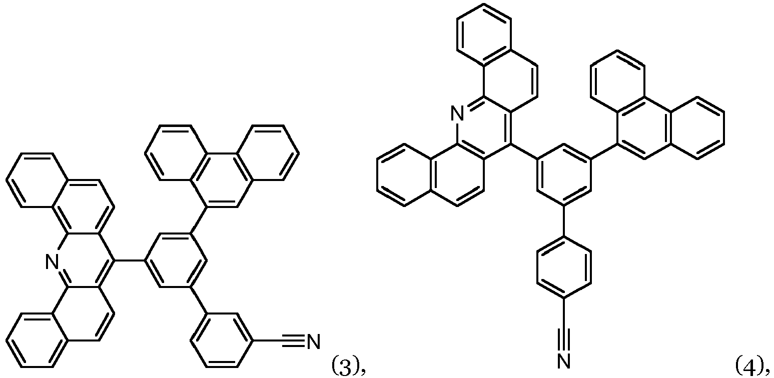

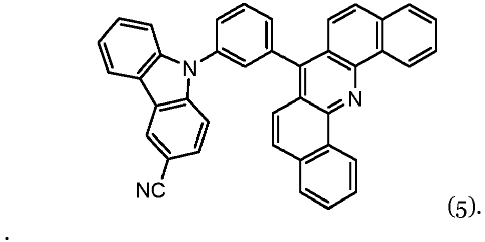

2. Organic semiconducting material according to claim 1, wherein one of R¹ to R¹³ is independently selected from the group consisting of CN-substituted C₆ to C₄₂ aryl, CN-substituted C₃ to C₄₂ heteroaryl, and the remaining R¹ to R¹³ are H.

3. Organic semiconducting material according to any of the claims 1 or 2, wherein R¹ to R¹³ are independently selected from the group consisting of substituted or unsubstituted C₆ to C₄₂ aryl and H, wherein the one or more substituent(s), if present, are independently selected from C₁ to C₁₈ alkyl.

4. Organic semiconducting material according to any of the claims 1 to 3, wherein R⁷ is CN-substituted C₆ to C₄₂ aryl, wherein further substituent(s), if present, are independently selected from C₁ to C₁₈ alkyl; and the remaining R¹ to R⁶ and R⁸ to R¹³ are H.

5. Organic semiconducting material according to any of the claims 1 to 4, wherein at least one of R¹ to R¹³ is C₁₂ to C₁₈ aryl substituted with at least one CN-group and optionally further substituted with at least one C₁ to C₄ alkyl group.

6. Organic semiconducting material according to claim 5, wherein R⁷ is C₁₂ to C₁₈ aryl substituted with a CN-group and optionally further substituted with at least one C₁ to C₄ alkyl group and the remaining R¹ to R⁶ and R⁸ to R¹³ are H.

7. Organic semiconducting material according to any of the preceding claims, wherein the compound (I) is a compound represented by one of the following structures A-1 to A-7

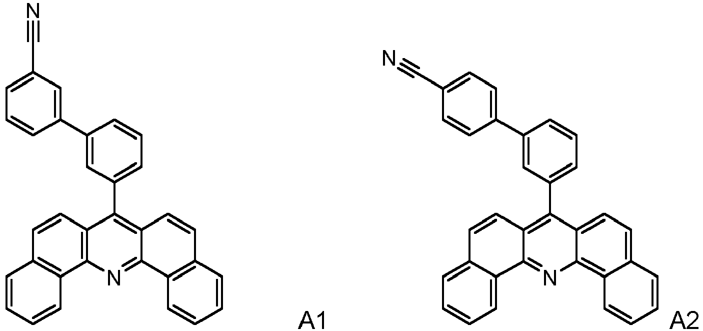

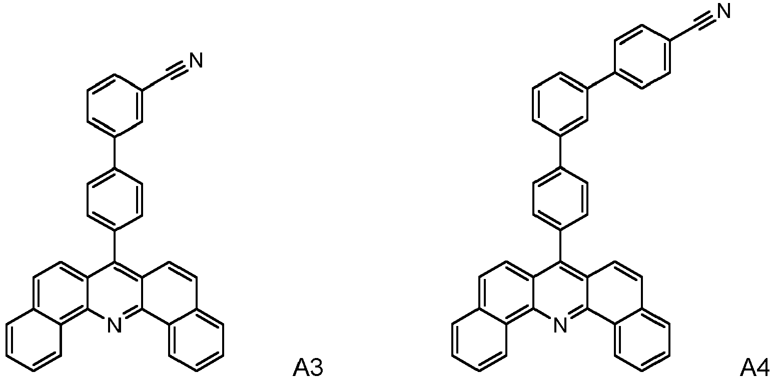

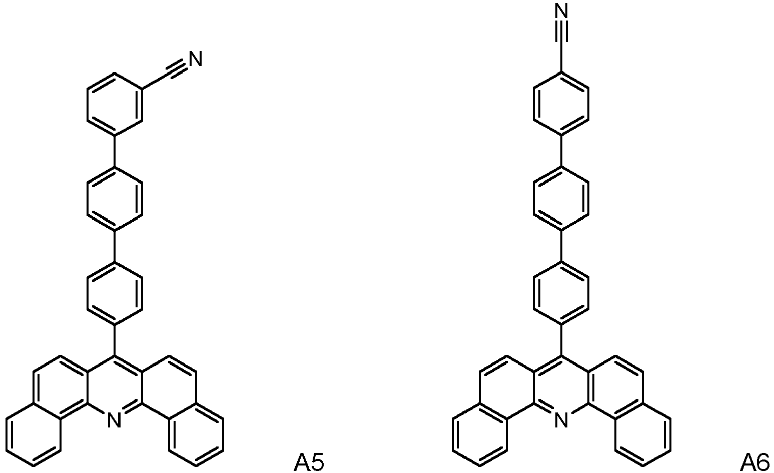

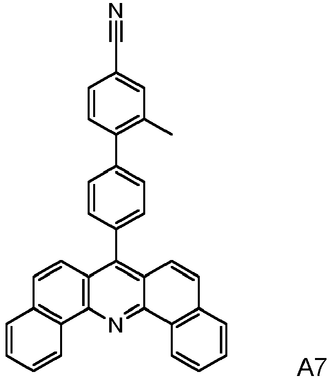

8. Organic semiconducting material according to any of the preceding claims, wherein the organic semiconducting material further comprises an n-dopant selected from the group consisting of metals, metal salts and metal complexes.

9. Organic electronic device comprising a first electrode, a second electrode and a semiconducting layer arranged between the first electrode and the second electrode, wherein the semiconducting layer comprises the organic semiconducting material according to any of the preceding claims.

10. Organic electronic device according to claim 9, wherein the semiconducting layer is an electron transport layer, an electron injection layer or an electron generating layer.

11. Organic electronic device according to claim 9 or 10, wherein the organic electronic device is an organic light emitting device, an organic photovoltaic device or an organic transistor.

12. Organic electronic device according to claim 11, wherein the organic light emitting device is an organic light emitting diode.

13. Display device comprising at least one, alternatively at least two organic light emitting devices according to claim 11 or 12.

14. Compound represented by the following formula (Ia) 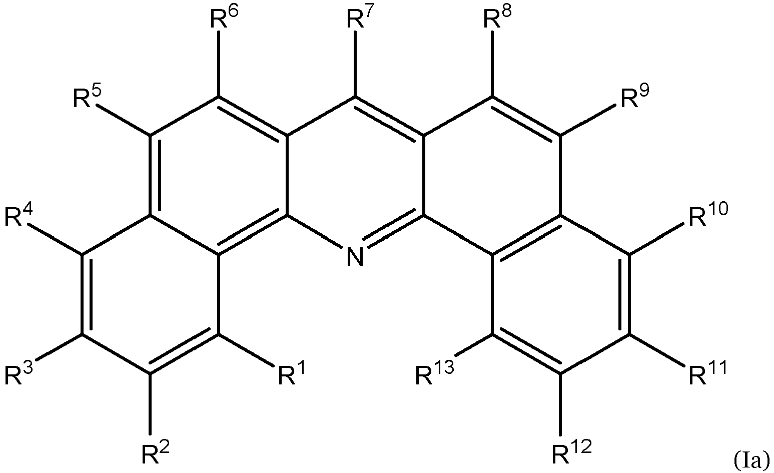
wherein R¹ to R¹³ are independently selected from the group consisting of H, D, F, Cl, Br, I, substituted or unsubstituted C₁ to C₁₈ alkyl, substituted or unsubstituted C₆ to C₄₂ aryl, substituted or unsubstituted C₃ to C₄₂ heteroaryl, wherein two or more groups selected from R¹ to R¹³ may be linked to form a nonaromatic ring;
wherein at least one of R¹ to R¹³ is a group substituted with at least one CN;
wherein the one or more substituent(s), if present in one of the groups R¹ to R¹³, are independently selected from the group consisting of D, F, Cl, Br, I, C₁ to C₁₈ alkyl, C₆ to C₃₆ aryl, C₆ to C₄₂ heteroaryl, R¹⁴R¹⁵P=O with R¹⁴ and R¹⁵ independently being C₁ to C₁₈ alkyl or C₆ to C₂₄ aryl, and CN, wherein compounds represented by the following formulas (1) to (22) are excluded

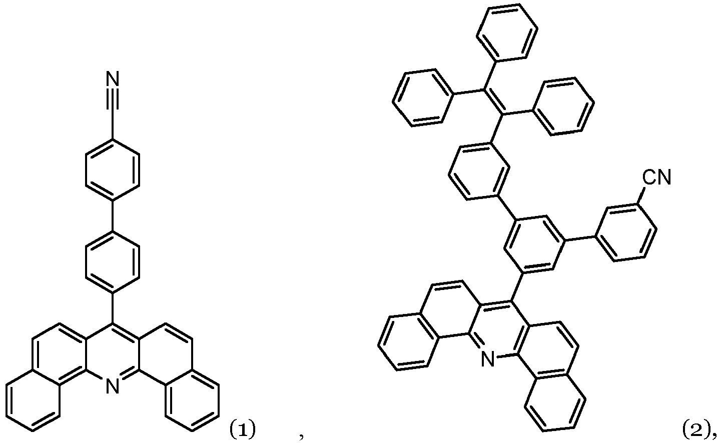

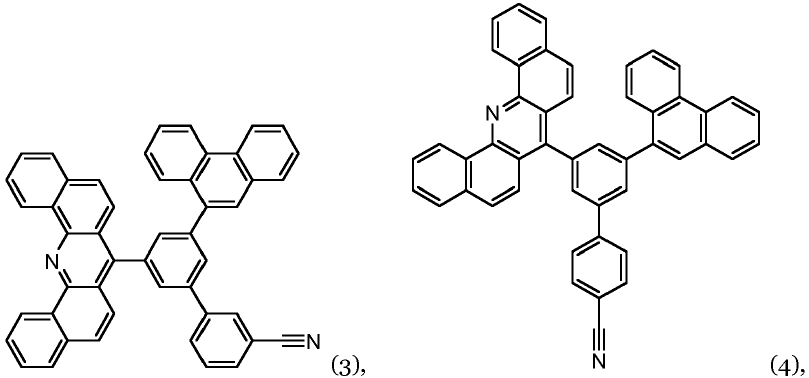

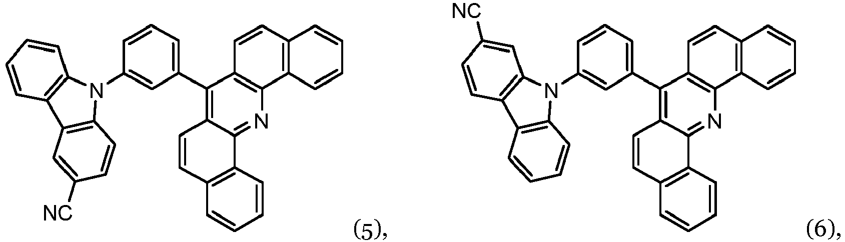

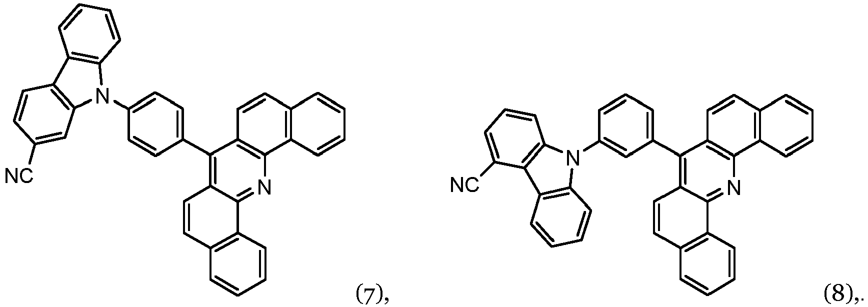

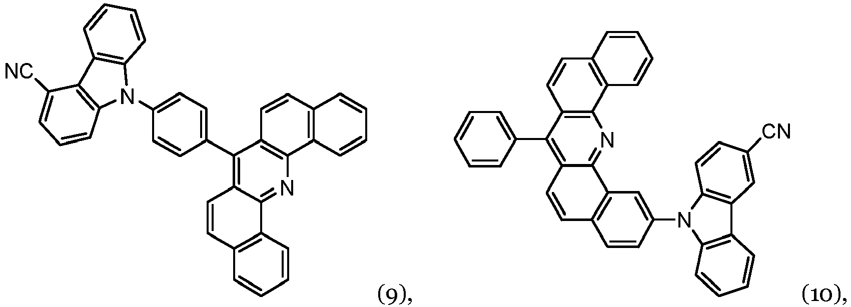

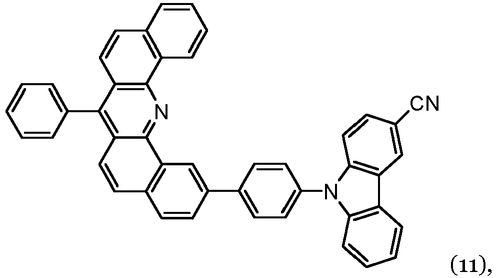

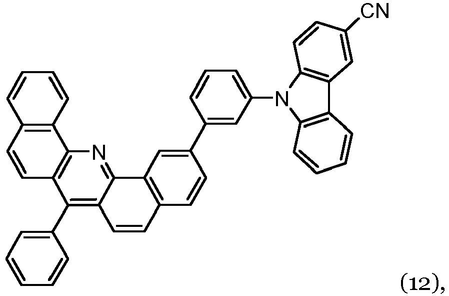

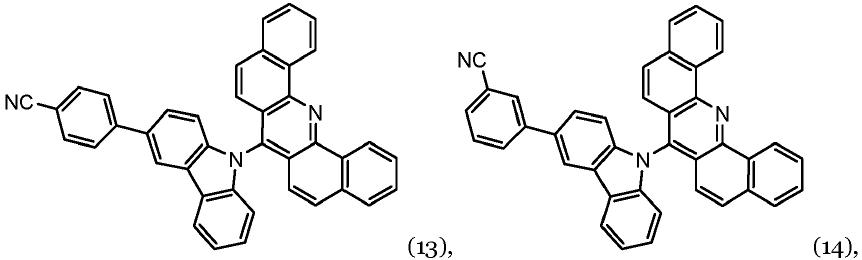

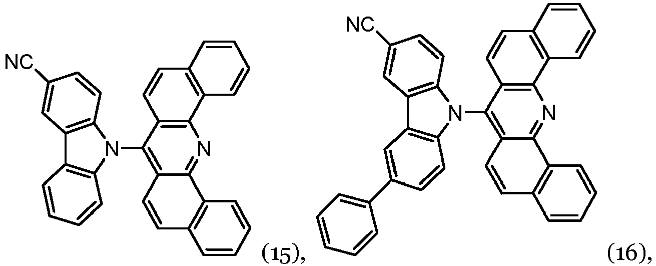

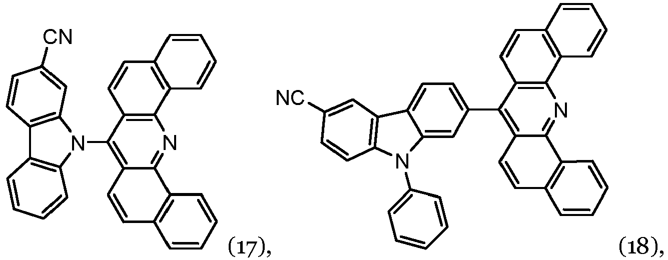

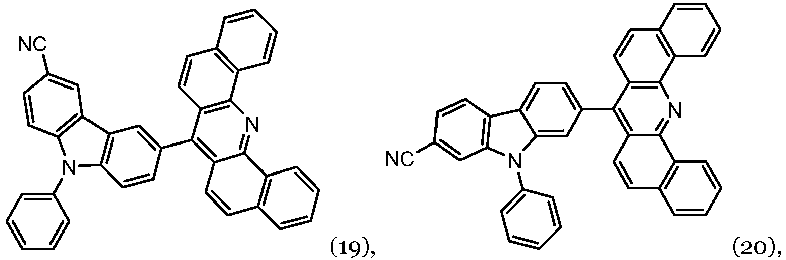

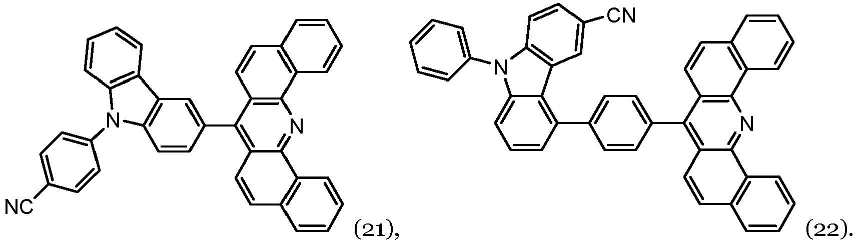

## Patentansprüche

1. Organisches halbleitendes Material, umfassend eine durch die folgende Formel (Ia) dargestellte Verbindung 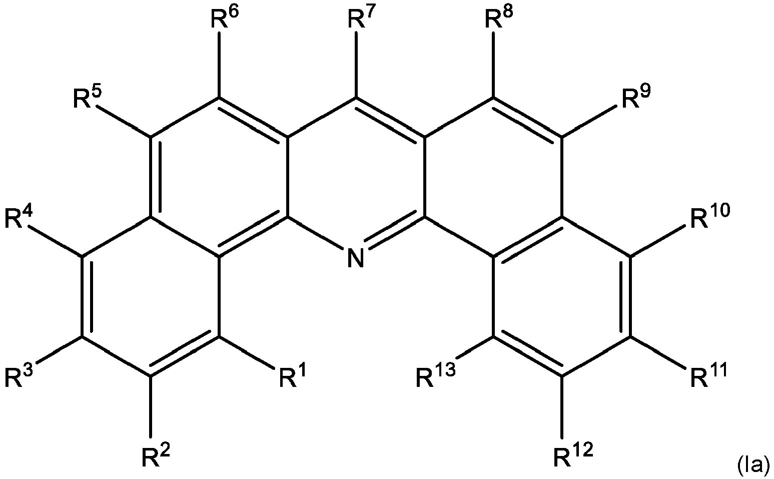
wobei R¹ bis R¹³ unabhängig ausgewählt sind aus der Gruppe bestehend aus H, D, F, Cl, Br, I, substituiertem oder unsubstituiertem C₁- bis C₁₈-Alkyl, substituiertem oder unsubstituiertem C₆- bis C₄₂-Aryl, substituiertem oder unsubstituiertem C₃-bis C₄₂-Heteroaryl, wobei zwei oder mehr aus R¹ bis R¹³ ausgewählte Gruppen verknüpft sein können, um einen nichtaromatischen Ring zu bilden;
wobei mindestens eines von R¹ bis R¹³ eine mit mindestens einem CN substituierte Gruppe ist;
wobei der eine oder die mehreren Substituenten, falls in einer der Gruppen R¹ bis R¹³ vorhanden, unabhängig ausgewählt sind aus der Gruppe bestehend aus D, F, Cl, Br, I, C₁- bis C₁₈-Alkyl, C₆- bis C₃₆-Aryl, C₆- bis C₄₂-Heteroaryl, R¹⁴R¹⁵P=O, wobei R¹⁴ und R¹⁵ unabhängig C₁- bis C₁₈-Alkyl oder C₆- bis C₂₄-Aryl sind, und CN,
wobei die durch die folgenden Formeln (1) bis (5) dargestellten Verbindungen ausgeschlossen sind

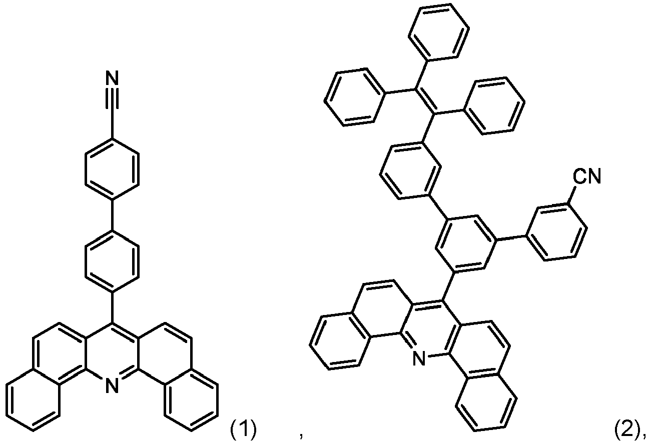

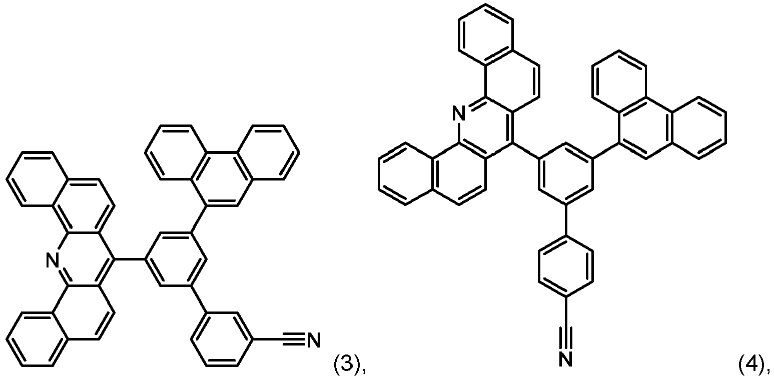

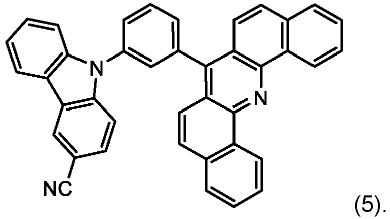

2. Organisches halbleitendes Material nach Anspruch 1, wobei eines von R¹ bis R¹³ unabhängig ausgewählt ist aus der Gruppe bestehend aus CN-substituiertem C₆- bis C₄₂-Aryl, CN-substituiertem C₃- bis C₄₂-Heteroaryl, und die übrigen R¹ bis R¹³ H sind.

3. Organisches halbleitendes Material nach einem der Ansprüche 1 oder 2, wobei R¹ bis R¹³ unabhängig ausgewählt sind aus der Gruppe bestehend aus substituiertem oder unsubstituiertem C₆- bis C₄₂-Aryl und H, wobei der eine oder die mehreren Substituenten, falls vorhanden, unabhängig ausgewählt sind aus C₁- bis C₁₈-Alkyl.

4. Organisches halbleitendes Material nach einem der Ansprüche 1 bis 3, wobei R⁷ CNsubstituiertes C₆- bis C₄₂-Aryl ist, wobei (ein) weitere(r) Substituent(en), falls vorhanden, unabhängig ausgewählt sind aus C₁- bis C₁₈-Alkyl; und die übrigen R1 bis R⁶ und R⁸ bis R¹³ H sind.

5. Organisches halbleitendes Material nach einem der Ansprüche 1 bis 4, wobei mindestens eines von R¹ bis R¹³ C₁₂- bis C₁₈-Aryl substituiert mit mindestens einer CN-Gruppe und gegebenenfalls ferner substituiert mit mindestens einer C₁- bis C₄-Alkylgruppe ist.

6. Organisches halbleitendes Material nach Anspruch 5, wobei R⁷ C₁₂- bis C₁₈-Aryl substituiert mit einer CN-Gruppe und gegebenenfalls ferner substituiert mit mindestens einer C₁- bis C₄-Alkylgruppe ist und die übrigen R¹ bis R⁶ und R⁸ bis R¹³ H sind.

7. Organisches halbleitendes Material nach einem der vorhergehenden Ansprüche, wobei die Verbindung (I) eine durch eine der folgenden Strukturen A-1 bis A-7 dargestellte Verbindung ist

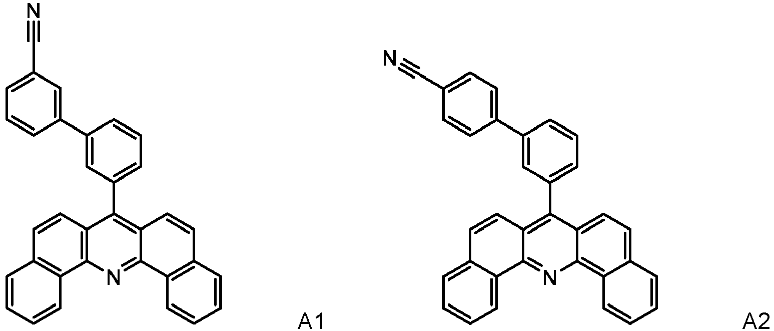

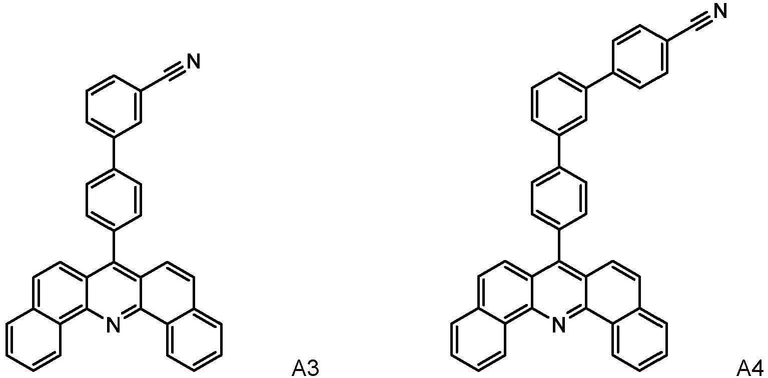

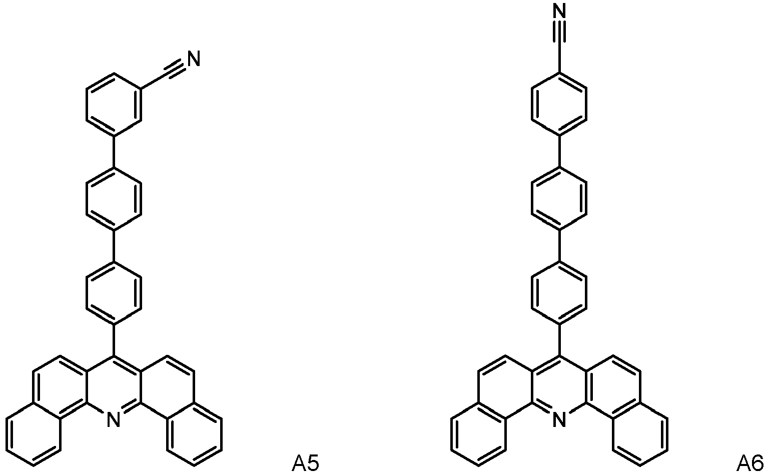

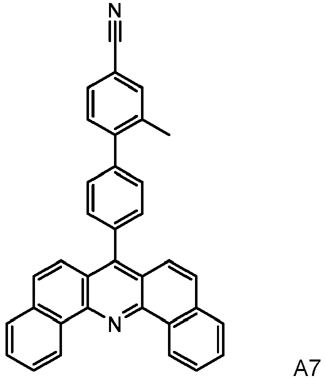

8. Organisches halbleitendes Material nach einem der vorhergehenden Ansprüche, wobei das organische halbleitende Material ferner ein n-Dotiermittel ausgewählt aus der Gruppe bestehend aus Metallen, Metallsalzen und Metallkomplexen umfasst.

9. Organische elektronische Vorrichtung, umfassend eine erste Elektrode, eine zweite Elektrode und eine halbleitende Schicht, die zwischen der ersten Elektrode und der zweiten Elektrode angeordnet ist, wobei die halbleitende Schicht das organische halbleitende Material nach einem der vorhergehenden Ansprüche umfasst.

10. Organische elektronische Vorrichtung nach Anspruch 9, wobei die halbleitende Schicht eine Elektronentransportschicht, eine Elektroneninjektionsschicht oder eine elektronenerzeugende Schicht ist.

11. Organische elektronische Vorrichtung nach Anspruch 9 oder 10, wobei die organische elektronische Vorrichtung eine organische lichtemittierende Vorrichtung, eine organische photovoltaische Vorrichtung oder ein organischer Transistor ist.

12. Organische elektronische Vorrichtung nach Anspruch 11, wobei die organische lichtemittierende Vorrichtung eine organische Leuchtdiode ist.

13. Anzeigevorrichtung, umfassend mindestens eine, alternativ mindestens zwei organische lichtemittierende Vorrichtungen nach Anspruch 11 oder 12.

14. Durch die folgende Formel (Ia) dargestellte Verbindung 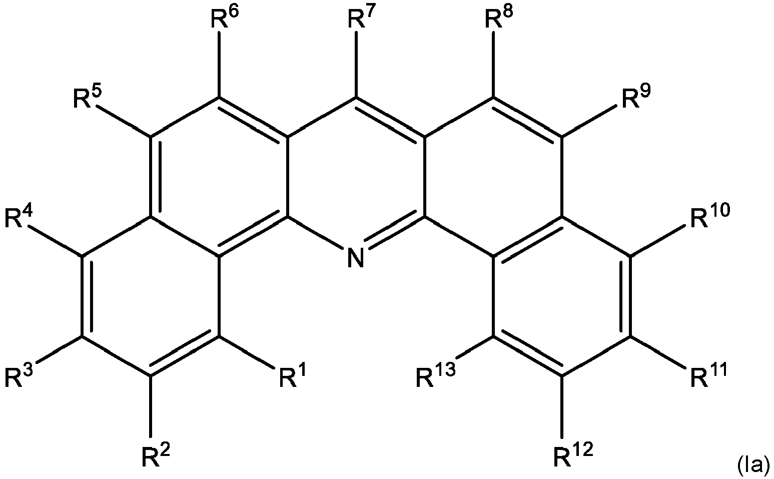
wobei R¹ bis R¹³ unabhängig ausgewählt sind aus der Gruppe bestehend aus H, D, F, Cl, Br, I, substituiertem oder unsubstituiertem C₁- bis C₁₈-Alkyl, substituiertem oder unsubstituiertem C₆- bis C₄₂-Aryl, substituiertem oder unsubstituiertem C₃- bis C₄₂-Heteroaryl, wobei zwei oder mehr aus R¹ bis R¹³ ausgewählte Gruppen verknüpft sein können, um einen nichtaromatischen Ring zu bilden;
wobei mindestens eines von R¹ bis R¹³ eine mit mindestens einem CN substituierte Gruppe ist;
wobei der eine oder die mehreren Substituenten, falls in einer der Gruppen R¹ bis R¹³ vorhanden, unabhängig ausgewählt sind aus der Gruppe bestehend aus D, F, Cl, Br, I, C₁- bis C₁₈-Alkyl, C₆- bis C₃₆-Aryl, C₆- bis C₄₂-Heteroaryl, R¹⁴R¹⁵P=O, wobei R¹⁴ und R¹⁵ unabhängig C₁- bis C₁₈-Alkyl oder C₆- bis C₂₄-Aryl sind, und CN, wobei durch die folgenden Formeln (1) bis (22) dargestellte Verbindungen ausgeschlossen sind

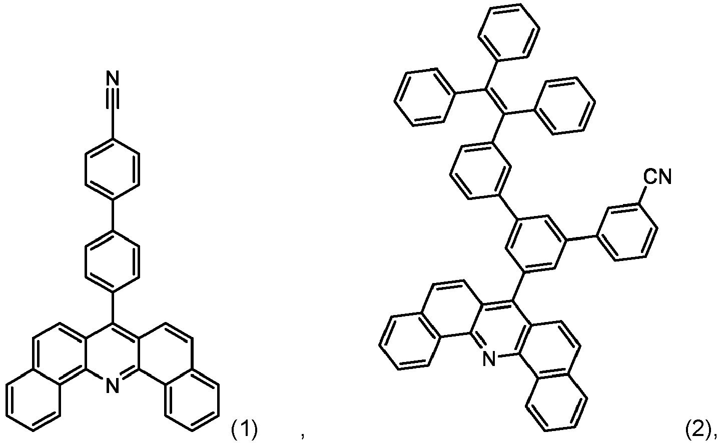

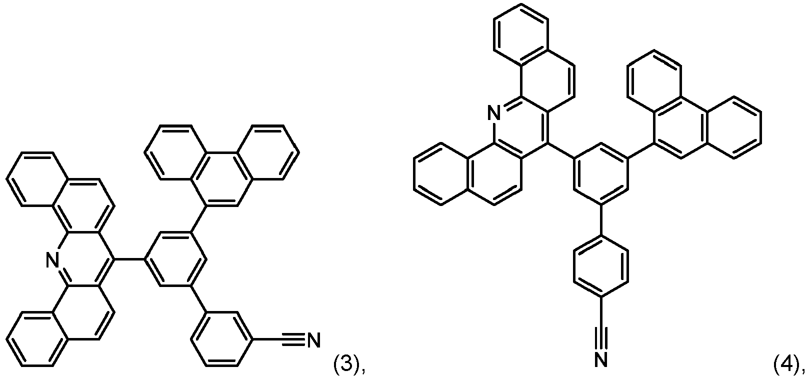

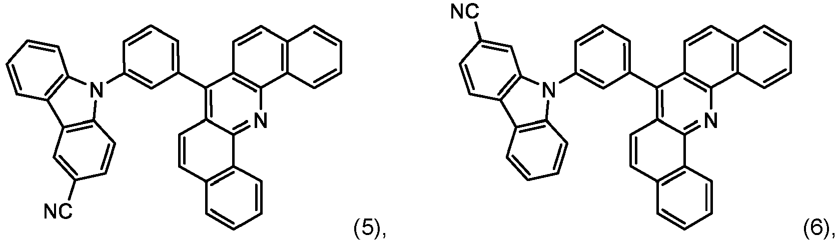

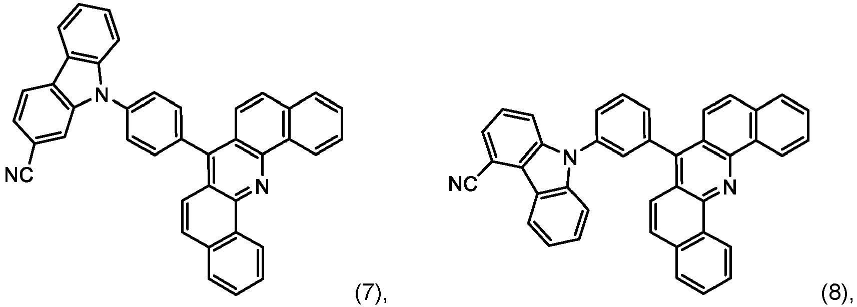

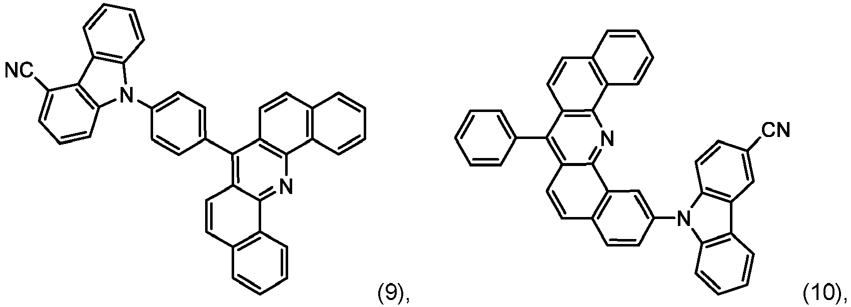

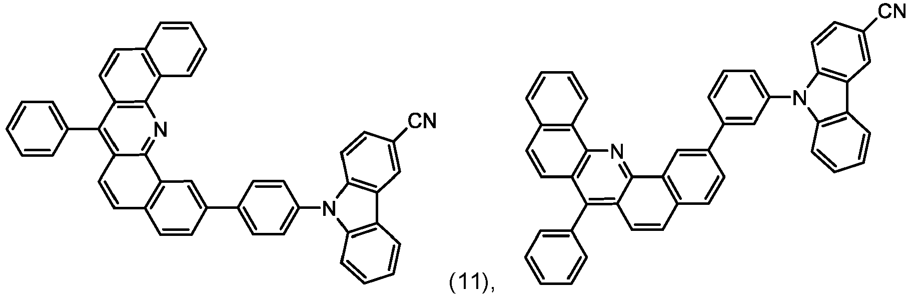

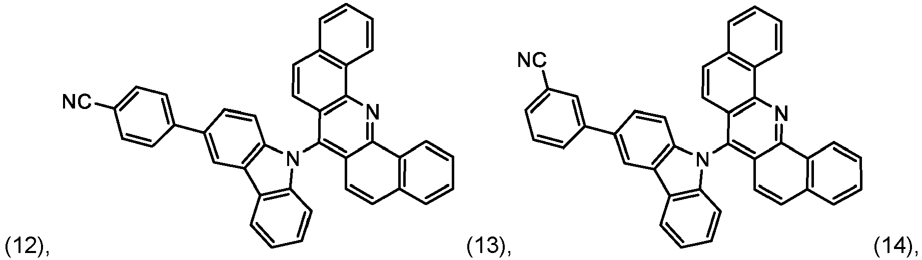

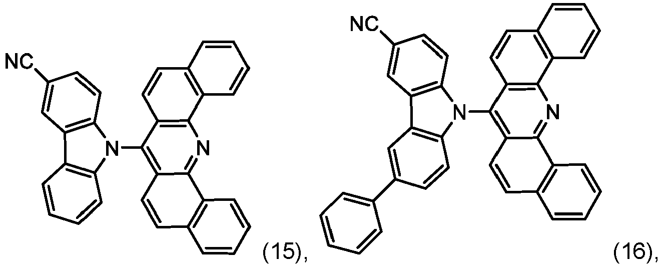

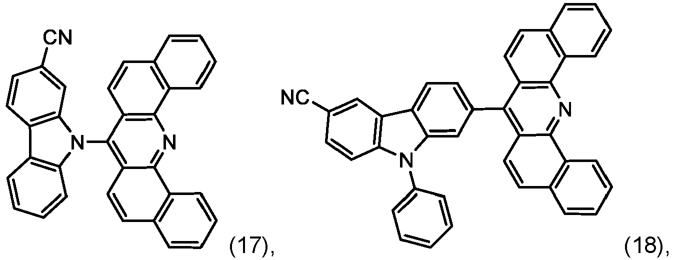

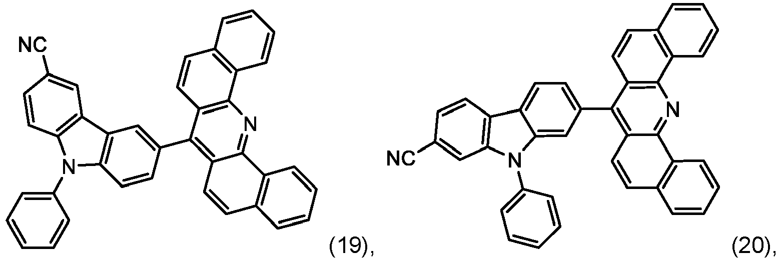

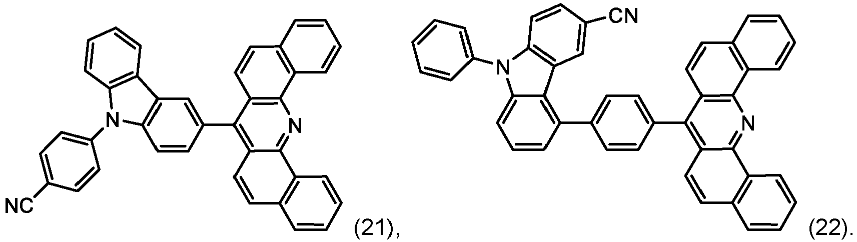

## Revendications

1. Matériau organique semi-conducteur comprenant un composé représenté par la formule suivante (la) 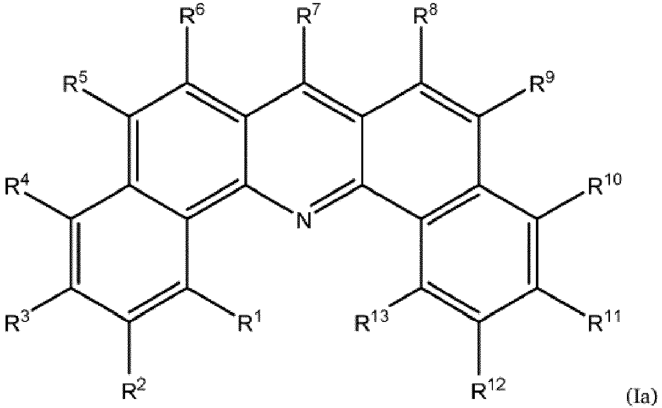
dans laquelle R¹ à R¹³ sont indépendamment choisis dans le groupe constitué par H, D, F, Cl, Br, I, un alkyle en C₁ à C₁₈ substitué ou non substitué, un aryle en C₆ à C₄₂ substitué ou non substitué, un hétéroaryle en C₃ à C₄₂ substitué ou non substitué, dans laquelle au moins deux groupes choisis parmi R¹ à R¹³ peuvent être liés afin de former un cycle non aromatique ;
dans laquelle au moins un parmi R¹ à R¹³ est un groupe substitué par au moins un CN ; dans laquelle l'un ou plusieurs substituants, si présent dans un des groupes R¹ à R¹³, sont indépendamment choisis dans le groupe constitué par D, F, Cl, Br, I, un alkyle en C₁ à C₁₈, un aryle en C₆ à C₃₆, un hétéroaryle en C₆ à C₄₂, R¹⁴R¹⁵P=O avec R¹⁴ et R¹⁵ représentant indépendamment un alkyle en C₁ à C₁₈, un aryle en C₆ à C₂₄, et CN,
dans lequel les composés représentés par les formules (1) à (5) suivantes sont exclus

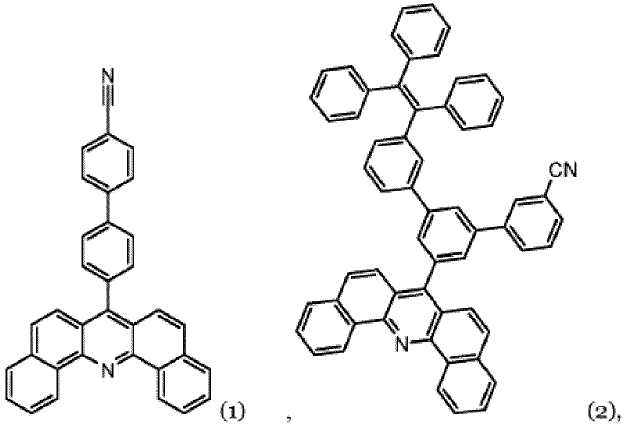

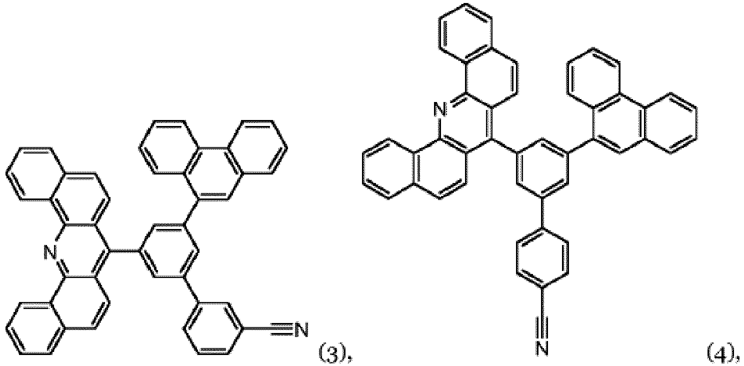

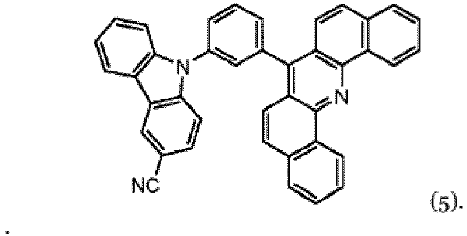

2. Matériau organique semi-conducteur selon la revendication 1, dans lequel l'un des R¹ à R¹³ est indépendamment choisi dans le groupe constitué par un aryle en C₆ à C₄₂ substitué par un CN, un hétéroaryle en C₃ à C₄₂ substitué par un CN, et les R¹ à R¹³ restants sont H.

3. Matériau organique semi-conducteur selon la revendication 1 ou la revendication 2, dans lequel R¹ à R¹³ sont indépendamment choisis dans le groupe constitué par un aryle en C₆ à C₄₂ substitué ou non substitué et H, dans lequel l'un ou plusieurs substituants, si présents, sont indépendamment choisis parmi un alkyle en C₁ à C₁₈.

4. Matériau organique semi-conducteur selon l'une quelconque des revendications 1 à 3, dans lequel R⁷ représente un aryle en C₆ à C₄₂ substitué par un CN, dans lequel d'autres substituant, si présents, sont indépendamment choisis parmi un alkyle en C₁ à C₁₈ ; et les R¹ à R⁶ et R⁸ à R¹³ restants sont H.

5. Matériau organique semi-conducteur selon l'une quelconque des revendications 1 à 4, dans lequel au moins un des R¹ à R¹³ est un aryle en C₁₂ à C₁₈ substitué par au moins un groupe CN et éventuellement en outre substitué par au moins un groupe alkyle en C₁ à C₄.

6. Matériau organique semi-conducteur selon la revendication 5, dans lequel R⁷ est un aryle en C₁₂ à C₁₈ substitué par un groupe CN et éventuellement en outre substitué par au moins un groupe alkyle en C₁ à C₄ et les R¹ à R⁶ et R⁸ à R¹³ restants sont H.

7. Matériau organique semi-conducteur selon l'une quelconque des revendications précédentes, dans lequel le composé (I) est un composé représenté par l'une des structures A-1 à A-7 suivantes

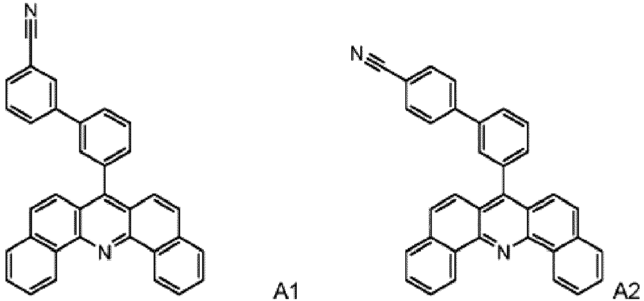

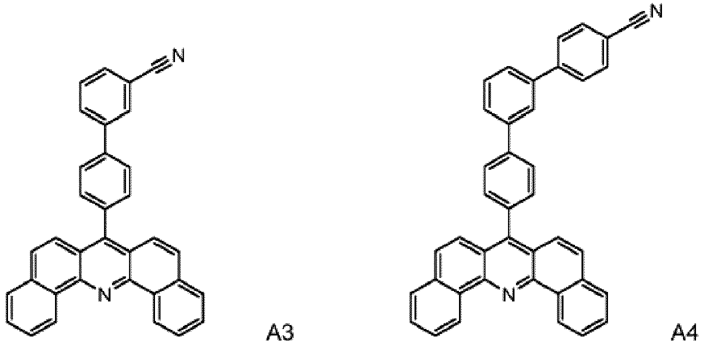

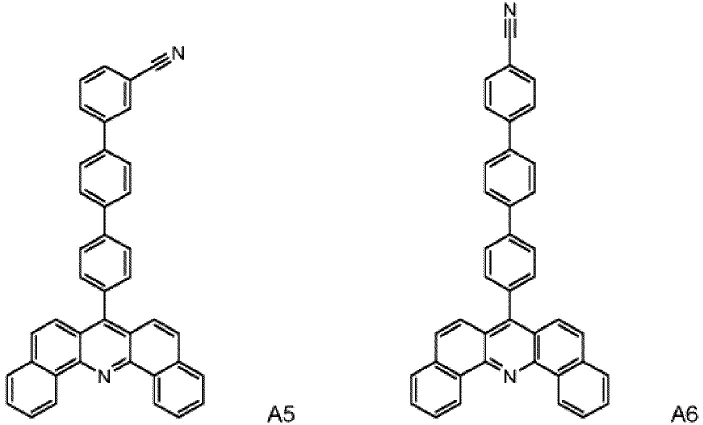

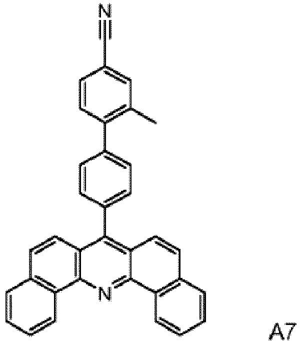

8. Matériau organique semi-conducteur selon l'une quelconque des revendications précédentes, dans lequel le matériau organique semi-conducteur comprend en outre un dopant NA choisis dans le groupe constitué par les métaux, les sels métalliques et les complexes métalliques.

9. Dispositif électronique organique comprenant une première électrode, une deuxième électrode et une couche semi-conductrice arrangée entre la première électrode et la deuxième électrode, dans lequel la couche semi-conductrice comprend le matériau organique semi-conducteur selon l'une quelconque des revendications précédentes.

10. Dispositif électronique organique selon la revendication 9, dans lequel la couche semi-conductrice est une couche de transport d'électrons, une couche d'injection d'électrons ou une couche de génération d'électrons.

11. Dispositif électronique organique selon la revendication 9 ou la revendication 10, dans lequel le dispositif électronique organique est un dispositif organique électroluminescent, un dispositif photovoltaïque organique ou un transistor organique.

12. Dispositif électronique organique selon la revendication 11, dans lequel le dispositif organique électroluminescent est une diode organique électroluminescente.

13. Dispositif d'affichage comprenant au moins un, alternativement au moins deux dispositifs organiques électroluminescents selon la revendication 11 ou la revendication 12.

14. Composé représenté par la formule (la) suivante 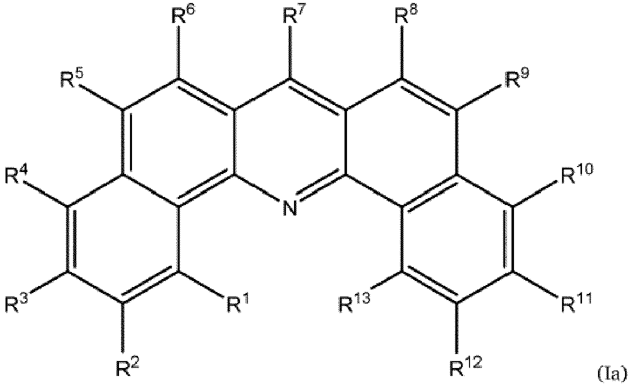
dans laquelle R¹ à R¹³ sont indépendamment choisis dans le groupe constitué par H, D, F, Cl, Br, I, un alkyle en C₁ à C₁₈ substitué ou non substitué, un aryle en C₆ à C₄₂ substitué ou non substitué, un hétéroaryle en C₃ à C₄₂ substitué ou non substitué, dans laquelle au moins deux groupes choisis parmi R¹ à R¹³ peuvent être liés afin de former un cycle non aromatique ;
dans laquelle au moins un parmi R¹ à R¹³ est un groupe substitué par au moins un CN ; dans laquelle l'un ou plusieurs substituants, si présenta dans un des groupes R¹ à R¹³, sont indépendamment choisis dans le groupe constitué par D, F, Cl, Br, I, un alkyle en C₁ à C₁₈, un aryle en C₆ à C₃₆, un hétéroaryle en C₆ à C₄₂, R¹⁴R¹⁵P=O avec R¹⁴ et R¹⁵ représentant indépendamment un alkyle en C₁ à C₁₈, un aryle en C₆ à C₂₄, et CN, dans lequel les composés représentés par les formules (1) à (22) suivantes sont exclus
